# EUROPEAN PATENT APPLICATION

(11) **EP 2 471 801 A2**
(43) Date of publication of application: **04.07.2012**
(21) Application number: 10812299.5
(22) Date of filing: 26.08.2010
(51) Int. Cl.: C07H 1/06, C07H 15/04, C07H 11/04

(54) **LIPOTEICHOIC ACID-DERIVED GLYCOLIPIDS, AND COMPOSITIONS COMPRISING SAME**

(30) Priority: 26.08.2009 KR 20090079482
(71) Applicant: RNA Inc., Gyeonggi-do 446-906 (KR)
(72) Inventor: CHUNG, Dae Kyun, Suwon-si Gyeonggi-do 442-190 (KR); JANG, Kyoung Soon, Seoul 151-056 (KR); KIM, Byung Gee, Seoul 137-070 (KR); KIM, Han Geun, Suwon-si Gyeonggi-do 442-844 (KR); KIM, Na Ra, Suwon-si Gyeonggi-do 443-471 (KR); LEE, Hea Young, Yongin-si Gyeonggi-do 446-701 (KR); JUNG, Bong Jun, Suwon-si Gyeonggi-do 443-390 (KR); KIM, Tae Rahk, Suwon-si Gyeonggi-do 443-470 (KR); JEONG, Ji Hye, Seosan-ri Chungcheongnam-do 356-851 (KR)
(74) Representative: Vossius & Partner
(86) International application number: PCT/KR2010/005755
(87) International publication number: WO 2011/025286

(57) **Abstract**

A lipoteichoic acid (LTA)-derived glycolipid, and a pharmaceutical, food or cosmetic composition and a vaccine adjuvant including the same are provided. The LTA-derived glycolipid can inhibit the production of inflammatory cytokines and thus have anti-inflammatory effects. Therefore, the pharmaceutical, food or cosmetic compositions including the LTA-derived glycolipid can be used to prevent and treat inflammatory diseases, and the LTA-derived glycolipid can be also used as the vaccine adjuvant.

## Description

### [Technical Field]

The present invention relates to a lipoteichoic acid-derived glycolipid, and a pharmaceutical, food or cosmetic composition and a vaccine adjuvant including the same.

### [Background Art]

Lipoteichoic acid (LTA) is one of the components present within the cell wall of gram-positive bacteria. LTA is generally classified into two structures: poly(glycerophosphate) having a glucose or D-alanine substituent and a glycolipid including a sugar unit and a fatty acid chain. It is known that the expressions of inflammatory cytokines such as TNF-α are regulated depending on the content of D-alanine in the poly(glycerophosphate) (Morath, S., A. Geyer, et al. 2001, Structure-function relationship of cytokine induction by LTA from Staphylococcus aureus. J. Exp. Med. 193:393-397).

In particular, it was found that LTA isolated from *Staphylococcus aureus* (aLTA) causes septicemia in gram-positive bacteria, and aLTA is recognized by toll-like receptor 2 (TLR2) and cell membrane-associated CD14 of immunocytes such as macrophages to induce an increase in expression of inflammatory cytokines through the activities of NF-κB and MAP kinase.

However, LTA isolated from a lactobacillus (*Lactobacillus* sp.) has a different immune activity than aLTA (de Vos, W. J. 2005. Lipoteichoic acid in lactobacilli: D-alanine makes the differences. Proc. Natl. Acad. Sci. USA 102 (31):10763-4). That is, the aLTA functions to induce excessive inflammatory response from immunocytes, but the LTA isolated from the lactobacillus functions to suppress the inflammatory response of immunocytes. Such difference in the immune activity of LTA is considered to be derived from the structural difference between the two LTAs. Thus, it is very important to analyze a structure of LTA in an aspect of elucidating the functions of the LTA so as to control the immune activities.

### [Disclosure]

### [Technical Problem]

The present invention is directed to providing a glycolipid having anti-inflammatory effects, and a pharmaceutical, food or cosmetic composition and a vaccine adjuvant including the same.

### [Technical Solution]

One aspect of the present invention provides an LTA-derived glycolipid and a pharmaceutical, food or cosmetic composition and a vaccine adjuvant including the same.

### [Advantageous Effects]

The LTA-derived glycolipid according to the present invention shows an anti-inflammatory effect by suppressing the production of inflammatory cytokines, and thus the pharmaceutical, food and cosmetic compositions including the LTA-derived glycolipid can be used to prevent and treat inflammatory diseases, and the LTA-derived glycolipid can also be used as the vaccine adjuvant.

### [Description of Drawings]

FIG. 1 shows the ¹H, ¹³C and 2D-NMR spectra of LTA derived from *L. plantarum.*
FIG. 2 shows the COSY spectrum of LTA derived from *L. plantarum.*
FIG. 3 shows the HMQC spectrum of LTA derived from *L. plantarum* (A: α-methylene and methylene linked to olefin carbons, and B: anomeric protons of α-D-galactose and α-D-glucose).
FIG. 4 shows the GC/MS analysis results of an LTA hydrolysate.
FIG. 5 shows the MALDI-TOF MS spectrum of an LTA glycolipid (A: *S*. *aureus,* and B: *L. plantarum*).
FIG. 6 shows the MALDI-TOF MS spectra of an intact glycolipid and an O-acetylated glycolipid of (A) aLTA and (B) pLTA.
FIG. 7 shows the structure of a glycolipid ofpLTA.
FIG. 8 shows the tables listing the results obtained by analyzing molecular masses of pLTA glycolipids.
FIG. 9 shows the LTQ-Orbitrap FTMS spectra of (A) aLTA glycolipid and (B) pLTA glycolipid.
FIG. 10 shows the negative-ion collision-induced dissociation (CID) spectrum of the LTA glycolipid (A: dihexosyl-diacyl-glycerol, and B: trihexosyl-diacyl-glycerol).
FIG. 11 shows the tables listing the results obtained by analyzing molecular masses of aLTA glycolipids.
FIG. 12 shows the MALDI-TOF MS spectrum of a deacylated glycolipid of aLTA (a: intact glycolipid, b: NaOH-treated glycolipid, and c: Ca(OH)₂-treated glycolipid).
FIG. 13 shows the MALDI-TOF analysis results of glycolipid moieties of pLTA, dLTA, rLTA and sLTA.
FIG. 14 shows the experimental results showing the abilities of intact aLTA and pLTA, and glycolipid and poly(glycerophosphate) derived from the intact aLTA and pLTA to induce expression ofTNF-α.
FIG. 15 shows the experimental results showing the abilities of intact aLTA, de-alanine aLTA and de-acyl aLTA to suppress expression of TNF-α
FIG. 16 shows the experimental results showing the abilities of intact pLTA, de-alanine pLTA and de-acyl pLTA to suppress expression of TNF-α.
FIG. 17 shows that an expression level of TNF-α is decreased in cells pre-treated with a pLTA-derived glycolipid.
FIG. 18 shows the effects of glycolipids isolated from different LTAs of suppressing expression of TNF-α.
FIG. 19 shows the effects of *Shigella flexneri* peptidoglycan-induced TNF-α and IL-1β of suppressing expression of TNF-α according to pLTA treatment (A: expression measurement of TNF-α, B: expression measurement of IL-1β, and C: expression measurement of TNF-α after treatment with polymyxin B)
FIG. 20 shows (A) a change in expression level of NOD2 mRNA, (B) a change in expression level of NOD2 protein, and (C) a change in activity of NF- κB according to pLTA treatment for *S. flexneri* peptidoglycan-induced inflammatory responses.
FIG. 21 shows the results obtained by measuring changes in expression levels of TNF-α according to pLTA treatment for *S. flexneri* peptidoglycan-induced inflammatory responses (A: THP-1 cells transformed with control siRNA, B: THP-1 cells transformed with TLR2 siRNA, C: BMM isolated from a normal mouse, and D: BMM isolated from a TLR2 knock-out mouse).
FIG. 22 shows the changes in activities of MAP kinase and NF-κ according to pLTA treatment (A: phosphor-ERK and phosphor-SAPK/JNK activities, B: IκB-α degradation activity, and C: NF-κB activities measured by an immunofluorescence staining method).
FIG. 23 shows the (A) viability of septicemia-induced mice peritoneally injected with PBS or pLTA, and (B) contents of TNF-α in blood.
FIG. 24 shows the distribution of IL-4 in (A) a skin lesion and (B) skin tissue of a DNCB-induced atopy mouse according to pLTA treatment.
FIG. 25 shows that an expression level of TNF-α is decreased in cells pre-treated with rLTA.
FIG. 26 shows the effects of various LTAs of suppressing expression of TNF-α.
FIG. 27 shows the effects of various LTAs of suppressing expression of TNF-α according to treatment with certain signal inhibitors.

### [Best Mode]

LTA derived from a lactobacillus functions to induce the anti-inflammatory activity that suppresses expression of inflammatory cytokines. The present inventors have found that LTA derived from the lactobacillus *Lactobacillus plantarum* (pLTA) had a different glycolipid structure than aLTA, which functions to induce the inflammatory response. In particular, they have found that pLTA itself and a glycolipid moiety obtained by removing a poly(glycerophosphate) moiety from the pLTA had an anti-inflammatory effect of suppressing expression of inflammatory cytokines. Therefore, the present invention was completed based on these facts. That is, a variety of similar glycolipid variants having the anti-inflammatory effect may be prepared based on the glycolipid structure of pLTA so that the variety of similar glycolipid variants can be effectively applied.

The present invention is directed to providing a glycolipid having 2 to 6 saturated or unsaturated C₁₀ to C₃₀ acyl chains bound to at least one of C₁, C₃, C₄ and C₆ positions dihexose or trihexose which is linked by an α-bond.

In this case, the dihexose or trihexose may be glucose, galactose, mannose or fructose, and glucose or galactose may be preferred.

Also, the glycolipid may include at least one unsaturated acyl chain.

According to one exemplary embodiment of the present invention, the glycolipid may be a glycolipid having a structure represented by the following Formula I.

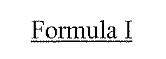

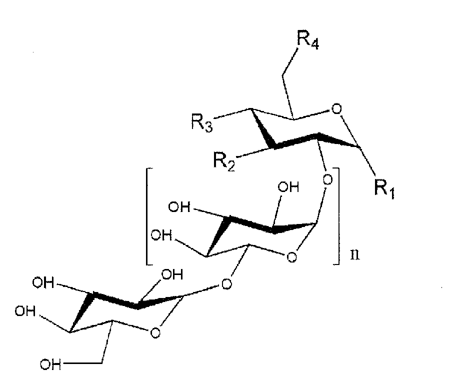

In Formula I, R₁ to R₄ may each independently represent a hydroxyl group, 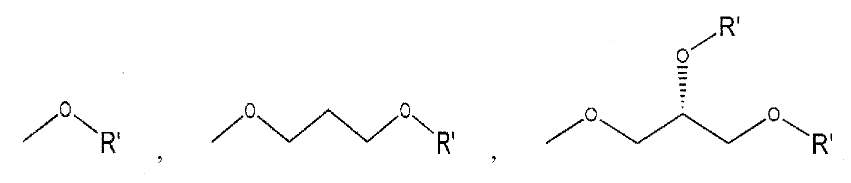 or 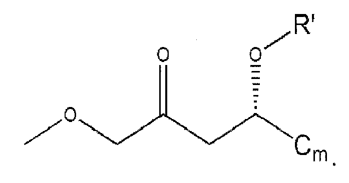 In this case, R' may each independently represent a saturated or unsaturated C₁₀ to C₃₀ acyl chain, and more particularly a saturated or unsaturated C₁₄ to C₂₆ acyl chain, a saturated or unsaturated C₁₄ to C₂₄ acyl chain, a saturated or unsaturated C₁₄ to C₂₂ acyl chain, a saturated or unsaturated C₁₄ to C₂₀ acyl chain, a saturated or unsaturated C₁₄ to C₁₈ acyl chain, a saturated or unsaturated C₁₆ to C₂₀ acyl chain, or a saturated or unsaturated C₁₆ to C₁₈ acyl chain. n may be 0 or 1, Cₘ may be a C₄ to C₃₀ alkyl group, and more particularly a C₆ to C₂₆ alkyl or C₁₀ to C₂₄ alkyl group, and the total number of the acyl chains in the glycolipid may be in a range of 2 to 6, more particularly, 2 to 4 or 2 to 3.

More particularly, R₁ in Formula I may be 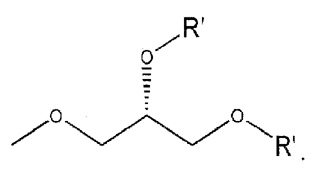 In this case, R' may each independently represent a saturated or unsaturated C₁₀ to C₃₀ acyl chain, and, more particularly, a saturated or unsaturated C₁₄ to C₂₆ acyl chain, a saturated or unsaturated C₁₄ to C₂₄ acyl chain, a saturated or unsaturated C₁₄ to C₂₂ acyl chain, a saturated or unsaturated C₁₄ to C₂₀ acyl chain, a saturated or unsaturated C₁₄ to C₁₈ acyl chain, a saturated or unsaturated C₁₆ to C₂₀ acyl chain, or a saturated or unsaturated C₁₆ to C₁₈ acyl chain, R₂ to R₄ may represent a hydroxyl group, n may be 1, and the glycolipid may include at least one acyl chain having 1 to 14 double bonds, and, more particularly, 1 to 10 double bonds, 1 to 8 double bonds, 1 to 6 double bonds, 1 to 4 double bonds, 1 to 3 double bonds or 1 to 2 double bonds.

More particularly, R₁ in Formula I may also be 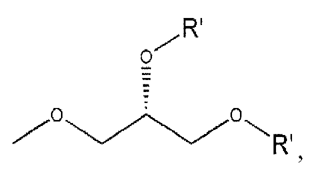 provided that one of R₂, R₃ and R₄ may be 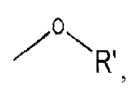 and the others may represent a hydroxyl group. In this case, R' may each independently represent a saturated or unsaturated C₁₀ to C₃₀ acyl chain, and, more particularly, a saturated or unsaturated C₁₄ to C₂₆ acyl chain, a saturated or unsaturated C₁₄ to C₂₄ acyl chain, a saturated or unsaturated C₁₄ to C₂₂ acyl chain, a saturated or unsaturated C₁₄ to C₂₀ acyl chain, a saturated or unsaturated C₁₄ to C₁₈ acyl chain, a saturated or unsaturated C₁₆ to C₂₀ acyl chain, or a saturated or unsaturated C₁₆ to C₁₈ acyl chain, n may be 1, and the glycolipid may include at least one acyl chain having 1 to 14 double bonds, more particularly, 1 to 10 double bonds, 1 to 8 double bonds, 1 to 6 double bonds, 1 to 4 double bonds, 1 to 3 double bonds, or 1 to 2 double bonds.

For example, the glycolipid of Formula I may be a glycolipid having a structure represented by the following Formula I-1 or I-2.

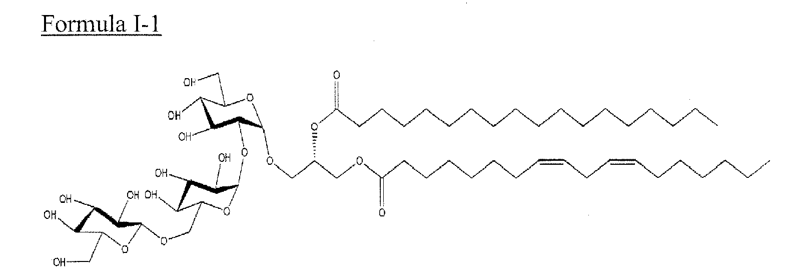

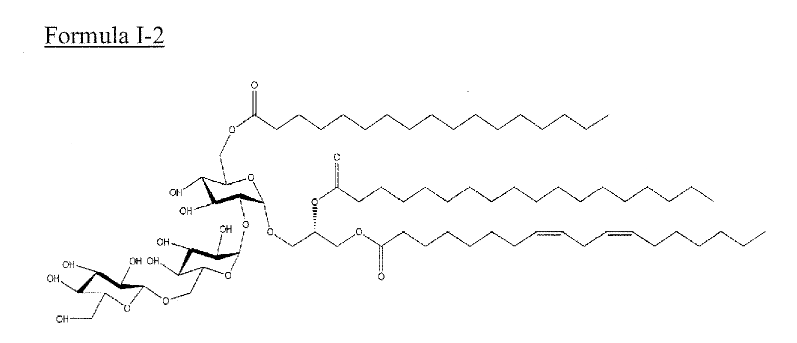

In this case, the positions of the double bonds indicated in Formula I-1 or I-2 may be varied.

According to another exemplary embodiment of the present invention, the glycolipid may be a lactobacillus-derived glycolipid, and the lactobacillus may be from, but is not limited to, the genus *Lactobacillus, Bifidobacterium, Streptococcus* or *Lactococcus.* For example, the genus *Lactobacillus* may be *Lactobacillus plantarum, Lactobacillus rhamnosus, Lactobacillus delbrueckii, Lactobacillus acidophilus, Lactobacillus gasseri, Lactobacillus johnsonni, Lactobacillus helveticus,* or *Lactobacillus casei,* the genus *Bifidobacterium* may be, for example, *Bifidobacterium bifido, Bifidobacterium longum, Bifidobacterium infantis,* or *Bifidobacterium animalis,* the genus *Streptococcus* may be, for exalple, *Streptococcus thermophilus,* and the genus *Lactococcus* may be, for example, *Lactococcus lactis.*

Among the LTAs, one having a well-defined structure is LTA (aLTA) derived from *S. aureus.* The aLTA derived from the pathogenic microorganism *S. aureus* functions to enhance expression of inflammatory cytokines by inducing the immunostimulatory activity. As shown in the following Formula III, the backbone of aLTA is known to be composed of gentiobiose having two glucoses linked by means of a β-bond [β1→6 linkage] and a poly(glycerophosphate) chain linked to C₆ of glucose by means of a 1,3-phosphodiester bond. Also, two saturated fatty acid chains (C_{15:0}) having on average 15 carbon atoms are linked to C₁ of glucose by means of an ester bond.

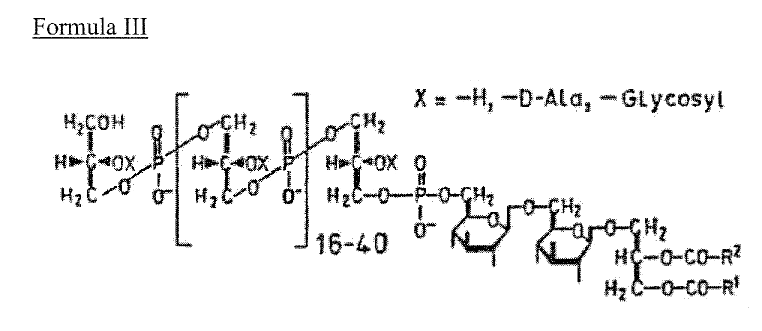

Meanwhile, pLTA derived from the lactobacillus functions to induce expression of inflammatory cytokines. Here, the structure of the pLTA glycolipid found in the present invention is **characterized in that** it has a backbone in which three hexose residues are linked by means of an α-bond, and poly(glycerophosphate) chains linked by means of a 1,3-phosphodiester bond are linked to a C₆ position of a terminal hexose residue opposite to the hexose residue to which a fatty acid acyl chain is linked. Also, the glycolipid of pLTA is **characterized in that** two or three fatty acid acyl chains are linked to a C₁ or C₆ position of the first hexose residue. More particularly, the pLTA-derived glycolipid may have a structure represented by Formula I-1 or I-2, but the position of double bonds in Formula I-1 or I-2 may be changed. Formula I-1 represents a Group I glycolipid of pLTA in which a saturated fatty acid acyl chain (C_{18:0}) having 18 carbon atoms and an unsaturated fatty acid acyl chain (C_{18:2}) having 18 carbon atoms are linked to a C₁ position of a hexose residue. Formula I-2 represents a Group II glycolipid of pLTA which further include a saturated fatty acid chain (C_{16:0}) having 16 carbon atoms, which is linked to a C₆ position of the hexose residue of the glycolipid represented by Formula I-1. Binding of hexose, the number of fatty acid chains and their binding positions, carbon atoms and the presence of unsaturated fatty acid play an important role in the ability of TLR2 to recognize LTA. Therefore, this structural characteristic serves as an important factor in inducing the anti-inflammatory activity of LTA.

In contrast to the fact that the expression of inflammatory cytokines such as TNF-α is regulated according to the content of D-alanine in the poly(glycerophosphate) of LTA as known in the art, according to the following exemplary embodiments, it can be seen that, in the case ofpLTA, a glycolipid structure including a fatty acid acyl chain plays a more important role in regulating the expression of inflammatory cytokines, compared to the D-alanine of the poly(glycerophosphate). Therefore, the present inventors have found that the pLTA-derived glycolipid itself shows an anti-inflammatory effect by suppressing the expression of an inflammatory cytokine, TNF-α. In addition to the pLTA-derived glycolipid, it was also confirmed that, in the case of *L. rhamnosus-derived* rLTA and *L. delbrueekii-derived* dLTA, each of which has a structure similar to the pLTA, only a glycolipid moiety also showed an anti-inflammatory effect by suppressing the expression of an inflammatory cytokine, TNF-α.

Also, the present invention is directed to providing a glycolipid in which a poly(glycerophosphate) is further linked to a C₆ position of a terminal hexose residue opposite to the hexose residue to which an acyl chain is linked.

According to one exemplary embodiment of the present invention, the glycolipid may be a glycolipid having a structure represented by the following Formula II.

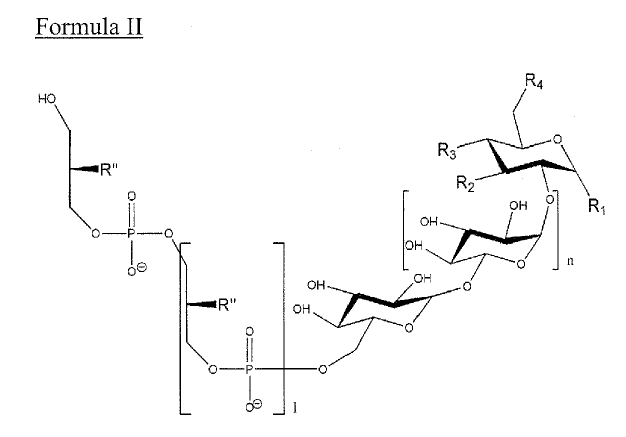

In Formula II, R₁ to R₄ may each independently represent a hydroxyl group, 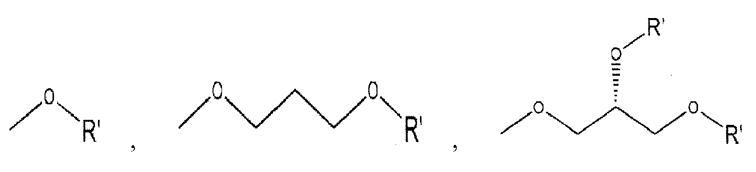 or 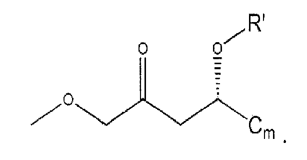 In this case, R' may each independently represent a saturated or unsaturated C₁₀ to C₃₀ acyl chain, and, more particularly, a saturated or unsaturated C₁₄ to C₂₆ acyl chain, a saturated or unsaturated C₁₄ to C₂₄ acyl chain, a saturated or unsaturated C₁₄ to C₂₂ acyl chain, a saturated or unsaturated C₁₄ to C₂₀ acyl chain, a saturated or unsaturated C₁₄ to C₁₈ acyl chain, a saturated or unsaturated C₁₆ to C₂₀ acyl chain, or a saturated or unsaturated C₁₆ to C₁₈ acyl chain. R" may each independently represent an N-acetylglucosamine, D-alanine or hydroxyl group. n may be 0 or 1, Cₘ may represent a C₄ to C₃₀ alkyl group, and, more particularly, a C₆ to C₂₆ alkyl or C₁₀ to C₂₄ alkyl group, and 1 may be in a range of 0 to 80, and, more particularly, 0 to 60, 0 to 40, or 0 to 20. The number of the acyl chains in the glycolipid may be in a range of 2 to 6, and, more particularly, 2 to 4, or 2 to 3.

More particularly, R₁ in Formula II may be 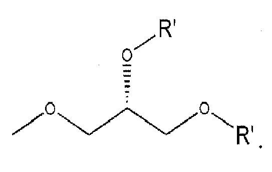 In this case, R' may each independently represent a saturated or unsaturated C₁₀ to C₃₀ acyl chain, and, more particularly, a saturated or unsaturated C₁₄ to C₂₆ acyl chain, a saturated or unsaturated C₁₄ to C₂₄ acyl chain, a saturated or unsaturated C₁₄ to C₂₂ acyl chain, a saturated or unsaturated C₁₄ to C₂₀ acyl chain, a saturated or unsaturated C₁₄ to C₁₈ acyl chain, a saturated or unsaturated C₁₆ to C₂₀ acyl chain, or a saturated or unsaturated C₁₆ to C₁₈ acyl chain, R" may each independently represent an N-acetylglucosamine, D-alanine or hydroxyl group, and R₂ to R₄ may each independently represent a hydroxyl group. n may be 1, 1 may be in a range of 0 to 80, more particularly 0 to 60, 0 to 40, or 0 to 20, and the glycolipid may include at least one acyl chain having 1 to 14 double bonds, and more particularly, 1 to 10 double bonds, 1 to 8 double bonds, 1 to 6 double bonds, 1 to 4 double bonds, 1 to 3 double bonds, or 1 to 2 double bonds.

More particularly, R₁ in Formula II may also be 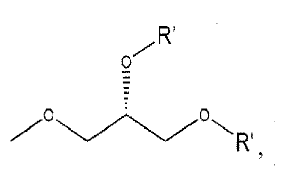 provided that one of R₂, R₃ and R₄ may be 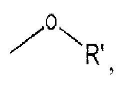 and the others may represent a hydroxyl group. In this case, R' may each independently represent a saturated or unsaturated C₁₀ to C₃₀ acyl chain, and, more particularly, a saturated or unsaturated C₁₄ to C₂₆ acyl chain, a saturated or unsaturated C₁₄ to C₂₄ acyl chain, a saturated or unsaturated C₁₄ to C₂₂ acyl chain, a saturated or unsaturated C₁₄ to C₂₀ acyl chain, a saturated or unsaturated C₁₄ to C₁₈ acyl chain, a saturated or unsaturated C₁₆ to C₂₀ acyl chain, or a saturated or unsaturated C₁₆ to C₁₈ acyl chain, R" may each independently represent an N-acetylglucosamine, D-alanine or hydroxyl group. n may be 1, 1 may be in a range of 0 to 80, more particularly, 0 to 60, 0 to 40, or 0 to 20, and the glycolipid may include at least one acyl chain having 1 to 14 double bonds, and, more particularly, 1 to 10 double bonds, 1 to 8 double bonds, 1 to 6 double bonds, 1 to 4 double bonds, 1 to 3 double bonds, or 1 to 2 double bonds.

For example, Formula II may be the following Formula 11-1 or II-2.

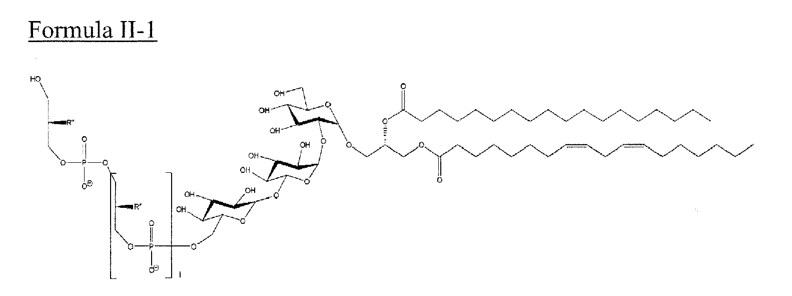

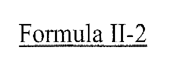

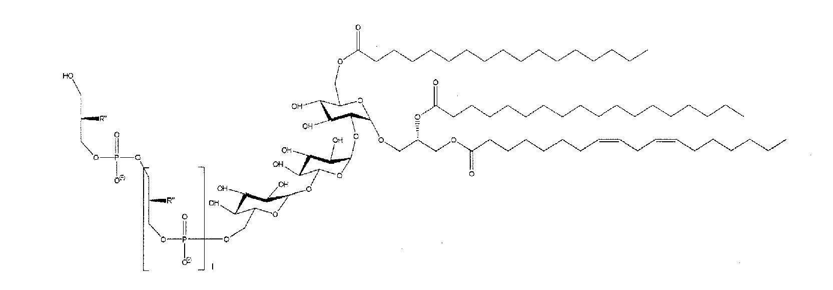

In Formula II-1 or II-2, R" may each independently represent an N-acetylglucosamine, D-alanine or hydroxyl group, and 1 may be in a range of 0 to 80, more particularly, 0 to 60, 0 to 40, or 0 to 20.

Also, the position of double bonds in Formula II-1 or II-2 may be changed.

According to one exemplary embodiment of the present invention, the glycolipid having the poly(glycerophosphate) further linked thereto may be a lactobacillus-derived LTA, and the lactobacillus may be from, but is not limited to, the genus *Lactobacillus, Bifidobacterium, Streptococcus* or *Lactococcus.* For example, the genus *Lactobacillus* may be *Lactobacillus plantarum, Lactobacillus rhamnosus, Lactobacillus delbrueckii, Lactobacillus acidophilus, Lactobacillus gasseri, Lactobacillus johnsonni, Lactobacillus helveticus,* or *Lactobacillus casei,* the genus *Bifidobacterium* may be, for example, *Bifidobacterium bifido, Bifidobacterium longum, Bifidobacterium infantis,* or *Bifidobacterium animalis,* the genus *Streptococcus* may be, for example, *Streptococcus thermophilus,* and the genus *Lactococcus* may be, for example, *Lactococcus lactis.*

Also, the present invention is directed to providing a pharmaceutical composition including the above-described glycolipid.

The glycolipid according to the present invention shows an anti-inflammatory effect of suppressing expression of an inflammatory cytokine, TNF-α, and IL-8 by suppressing the activity of NF-kB and the degradation of IkB-α, which are caused by stimulation of an endotoxin LPS, and suppressing the expression of TLR2, as described in the following Examples.

The pharmaceutical composition including the glycolipid according to the present invention may be formulated into an oral formulation, an external preparation, a suppository or a sterile injectable solution in the form of a powder, granule, tablet, capsule, suspension, emulsion, syrup or aerosol, depending on conventional methods, and may further include at least one suitable additive selected from the group consisting of an antibiotic, a carrier, an excipient and a diluent, which are widely used for preparation of pharmaceutical compositions.

More particularly, the carrier, excipient and diluent that may be used herein may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil. When the pharmaceutical composition is formulated into a preparation, the preparation may be formed using a diluent or excipient such as a filler, a bulking agent, a binding agent, a wetting agent, a disintegrating agent or a surfactant, which are widely used in the art. A solid preparation for oral administration may be a tablet, a pill, a powder, a granule or a capsule. Here, such a solid preparation may be formed by mixing at least one excipient, such as, for example, starch, calcium carbonate, sucrose, lactose or gelatin, with the extract. In addition to the simple excipient, lubricants such as magnesium stearate and talc may also be used herein. A liquid preparation for oral administration may be a suspension, a liquid for internal use, an emulsion or a syrup, and include various excipients, such as, for example, a wetting agent, a sweetening agent, an aromatic and a preservative in addition to the simple diluents such as water and liquid paraffin widely used in the art. A preparation for parenteral administration includes a sterile aqueous solution, a non-aqueous solvent, a suspension, an emulsion, a lyophilized preparation or a suppository. A vegetable oil such as propylene glycol, polyethylene glycol or olive oil, or an injectable ester such as ethyl oleate may be used as the non-aqueous solvent or suspension. A base of the suppository that may be used herein may include witepsol, macrogol, Tween 61, cacao butter, laurin butter or glycerogelatin.

In the pharmaceutical composition according to the present invention, the glycolipid may be included at a content of 0.01 to 99.9 parts by weight, 0.1 to 90 parts by weight, 1 to 80 parts by weight, or 10 to 80 parts by weight, based on 100 parts by weight of the pharmaceutical composition, but the present invention is not limited thereto. Therefore, the content of the glycolipid may be varied according to the conditions of a patient, and the kind and stage of a disease.

The glycolipid according to the present invention has an anti-inflammatory activity, and thus may be used to prevent and treat an inflammatory or immune-related disease. Therefore, the present invention is directed to providing a pharmaceutical composition for preventing and treating an inflammatory or immune-related disease including the above-described glycolipid, the use of the glycolipid for preparation of a medicine for preventing and treating an inflammatory or immune-related disease, and a method of preventing and treating an inflammatory disease including administering a therapeutically effective amount of the glycolipid to a subject.

More particularly, the inflammatory or immune-related disease may include septicemia, arteriosclerosis, bacteremia, a systemic inflammatory response syndrome, a multiple organ dysfunction syndrome, cancer, osteoporosis, paradentitis, systemic lupus erythematosus, rheumatoid arthritis, osteoarthritis, juvenile chronic arthritis, spondyloarthropathy, systemic scleroma, an idiopathic inflammatory disorder of muscle, Sjögren's syndrome, systemic vasculitis, sarcoidosis, autoimmune hemolytic anemia, autoimmune thrombocytopenia, thyroiditis, diabetes mellitus, an immune-mediated renal disease, a demyelinating disease in the central nervous system or peripheral nervous system, idiopathic demyelinating polyneuropathy, Guillain-Barre syndrome, chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease (IBD), ulcerative colitis, Crohn's disease, irritable bowel syndrome, gluten-irritable bowel disease, Whipple's disease, an autoimmune or immune-mediated skin disease, a bullous skin disease, erythema multiforme, contact dermatitis, psoriasis, an allergic disease, asthma, allergic rhinitis, atopic dermatitis, food sensitivity, urticaria, a pulmonary immune disease, eosinophilic pneumonia, idiopathic pulmonary fibrosis, hypersensitivity pneumonitis, a transplant-related disease, transplant rejection or a graft-versus-host disease.

According to one exemplary embodiment of the present invention, the glycolipid may be administered to a subject orally, rectally or by intravenous, intramuscular, subcutaneous, endometrial or intracerebroventricular injection.

A desirable dose of the glycolipid according to the present invention may be varied according to the conditions and body weight of a patient, the kind and stage of a disease, the type of a drug, and the route and period of administration, but may be properly selected by those skilled in the art. However, the glycolipid may be administered daily at a dose of 0.01 to 10,000 mg/kg, more particularly 0.1 to 10,000 mg/kg, and most particularly 0.1 to 1,000 mg/kg. Here, the glycolipid may be administered three times a day or administered in divided doses daily, but the present invention is not limited thereto.

According to the present invention, the term "subject" includes a human, an orangutan, a chimpanzee, a mouse, a rat, a dog, a bovine, a chicken, a pig, a goat or a sheep, but the present invention is not limited thereto.

In addition, the present invention is directed to providing a food composition including the glycolipid. The food composition may be used to prepare functional foods and conventional foods, which can have an effect of preventing and treating an inflammatory or immune-related disease selected from the group consisting of septicemia, arteriosclerosis, bacteremia, a systemic inflammatory response syndrome, a multiple organ dysfunction syndrome, cancer, osteoporosis, paradentitis, systemic lupus erythematosus, rheumatoid arthritis, osteoarthritis, juvenile chronic arthritis, spondyloarthropathy, systemic scleroma, an idiopathic inflammatory disorder of muscle, Sjögren's syndrome, systemic vasculitis, sarcoidosis, autoimmune hemolytic anemia, autoimmune thrombocytopenia, thyroiditis, diabetes mellitus, an immune-mediated renal disease, a demyelinating disease in the central nervous system or peripheral nervous system, idiopathic demyelinating polyneuropathy, Guillain-Barre syndrome, chronic inflammatory demyelinating polyneuropathy, a hepatobiliary disease, infectious or autoimmune chronic active hepatitis, primary biliary cirrhosis, granulomatous hepatitis, sclerosing cholangitis, IBD, ulcerative colitis, Crohn's disease, irritable bowel syndrome, gluten-irritable bowel disease, Whipple's disease, an autoimmune or immune-mediated skin disease, a bullous skin disease, erythema multiforme, contact dermatitis, psoriasis, an allergic disease, asthma, allergic rhinitis, atopic dermatitis, food sensitivity, urticaria, a pulmonary immune disease, eosinophilic pneumonia, idiopathic pulmonary fibrosis, hypersensitivity pneumonitis, a transplant-related disease, transplant rejection and a graft-versus-host disease.

According to one exemplary embodiment of the present invention, the food composition may further include at least one additive selected from the group consisting of organic acid, phosphate, an antioxidant, lactose, casein, dextrin, dextrose, sugar and sorbitol, but the present invention is not limited thereto. The organic acid may be citric acid, fumaric acid, adipic acid, lactic acid or malic acid, but the present invention is not limited thereto. The phosphate may be sodium phosphate, potassium phosphate, acid pyrophosphate or polyphosphate, but the present invention is not limited thereto. The antioxidant may be a natural antioxidant such as polyphenol, catechin, α-tocopherol, rosemary extract, licorice extract, chitosan, tannic acid or phytic acid, but the present invention is not limited thereto.

According to the present invention, the glycolipid may be included at a content of 0.01 to 99.9 parts by weight, and, more particularly, 1 to 90 parts by weight, or 30 to 80 parts by weight, based on 100 parts by weight of the food composition.

According to one exemplary embodiment of the present invention, a formulation of the food composition may also be in a solid, powder, granule, tablet, capsule or liquid type, but the present invention is not limited thereto.

The food composition including the glycolipid according to the present invention may be used to prepare foods such as snacks, sweets, ice cream products, dairy products, meat products, fish products, bean curds or jellies, edible oils and fats, noodles, teas, soft drinks, special nutrient foods, health supplement foods, seasonings, ice, ginseng products, kimchi curing products, dried shellfish, fruits, vegetables, dried products of fruits or vegetables, chopped products, fruit juices, vegetable juices, mixed juices thereof, chips, processed livestock foods, processed marine foods, processed dairy foods, fermented milk foods, soybean foods, cereal foods, microorganism-fermented foods, baked confections, condiments, processed meats, acidic beverages, licorice or herbs, but the present invention is not limited thereto. The types of foods include a solid, powder, granule, tablet, capsule, liquid or beverage type, but the present invention is not limited thereto.

According to another exemplary embodiment of the present invention, when a composition including the glycolipid is used to prepare a health functional food, the composition may be used at a content of 0.01 to 15% by weight, based on the entire weight of the food. When the composition is used to prepare a beverage, the composition may be used at a content of 0.02 to 10% by weight, or 0.3 to 1 % by weight, based on the entire weight of the food.

In addition to the glycolipid, the food composition according to the present invention may contain various additives such as a nutrient, a vitamin, a mineral (electrolyte), a flavoring agent such as a synthetic flavoring agent or a natural flavoring agent, a coloring agent, an improving agent (e.g., cheese, chocolate, etc.), pectic acid and salts thereof, alginic acid and salts thereof, an organic acid, a protective colloidal thickening agent, a pH controlling agent, a stabilizer, an antiseptic, glycerin, alcohol, or a carbonating agent used for carbonated beverages. Also, the food composition according to the present invention may include a fruit pulp for preparation of natural fruit juices, fruit juice beverages and vegetable beverages. A content of such an additive is not limited, but may be selected within a range of 0 to approximately 20 parts by weight, based on 100 parts by weight of the composition according to the present invention.

Also, the present invention is directed to providing a cosmetic composition including the glycolipid.

According to the present invention, the glycolipid may be included at a content of 0.01 to 99.9 parts by weight, and, more particularly, 1 to 90 parts by weight, or 30 to 80 parts by weight, based on 100 parts by weight of the cosmetic composition.

A formulation of cosmetics using the cosmetic composition according to the present invention is not limited, but may include, for example, a skin-softening lotion, a nutritional skin lotion, a massage cream, a nutrition cream, a pack, a jell, an essence, a lipstick, a makeup base, a foundation, a lotion, an ointment, a gel, a cream, a cleansing cream, a cleanser, a soap, a shampoo, a rinse, a treatment and a beauty lotion. Such cosmetics may include conventional components such as an aqueous vitamin, an oily vitamin, a high-molecular peptide, a high-molecular polysaccharide or a sphingolipid, and may be easily prepared using the techniques widely known in the art.

In addition to the components, the cosmetics may further include at least one additive selected from the group consisting of additives such as, for example, a vitamin, an amino acid, a protein, a surfactant, an emulsifying agent, a spice, a pigment, a stabilizing agent, an antiseptic, an antioxidant, a UV blocking agent, a pH controlling agent and a chelating agent, which are widely used for the cosmetics.

Also, the present invention is directed to providing a vaccine adjuvant consisting of or including the glycolipid.

The glycolipid according to the present invention may be used for various applications. One desirable application is to use the glycolipid as an immune stimulant or adjuvant for medicinal compositions including an immunogenic polynucleotide, a polypeptide, an antibody, a T-cell or an antigen-presenting cell (APC).

The adjuvant refers to a substrate that potentiates an immune response against antigens by inducing faster formation of antibodies by injection of a vaccine. The glycolipid according to the present invention may enhance an immune response against the antigens by affecting the expression of inflammatory cytokines and also affecting an immune action such as expression of adherent molecules. Also, since the glycolipid itself does not show immunogenicity, the glycolipid may be used as a vaccine adjuvant.

One immune response stimulated by the glycolipid according to the present invention may be by a Th1 or Th2 type, and thus the adjuvant according to the present invention may be designed to dominantly induce an immune response of the Th1 or Th2 type. A high concentration of Th1-type cytokines (for example, TNF-α, IFN-γ, IL-2 and IL-12) tends to aid in inducing a cell-mediated immune response against administered antigens. On the other hand, a high concentration of Th2-type cytokines (for example, IL-4, IL-5, IL-6, IL-10 and TNF-β) tends to aid in inducing a humoral immune response. After application of a vaccine including the glycolipid according to the present invention, a patient may maintain an immune response, including Th1- and Th2-type responses. When the response is induced by the Th1 type, the concentration of the Th1-type cytokines may be increased more than that of the Th2-type cytokines. The concentration of these cytokines may be easily measured using a standard assay. To investigate a cytokine family, see Mosmann and Coffman, Ann. Rev. Immunol. 7:145-173, 1989. CpG-containing oligonucleotides (including non-methylated CpG dinucleotides) generally function to induce a Th1 immune response. Such oligonucleotides are already known in the art, for example, as described in WO96/02555.

A desirable adjuvant used to induce a dominant Th1- or Th2-type response may include the glycolipid in combination of an aluminum salt.

### [Mode for Invention]

Hereinafter, the present invention will be described in further detail with reference to Examples to aid in understanding the present invention. However, the following Examples are provided for illustrative purposes only and are not intended to limit the scope of the present invention to the following Examples. Thus, the Examples of the present invention are provided to describe the present invention more completely, as apparent to a person of ordinary skill in the art.

### Experiment Examples

The Experiment Example provides an experimental example generally applied to each Example according to the present invention.

### Separation and purification of LTA- and LTA-derived glycolipid and poly(glycerophosphate)

Separation of LTA was performed using the method as reported in the art (Morath, S., A. Geyer, et al. 2001. Structure-function relationship of cytokine induction by LTA from Staphylococcus aureus. J. Exp. Med. 193(3):393-7.). 100 g of a cell (wet weight) was suspended in 400 ml of a 0.1 M sodium citrate buffer (pH 4.7), and then homogenized by sonication. An equivalent amount of n-butanol was added, mixed for 30 minutes, and then centrifuged to separate an aqueous layer including LTA. 15% n-propanol and a 0.1 M sodium citrate buffer (pH 4.7) were added to separate an LTA fraction, and then dialyzed through a semi-permeable membrane. Thereafter, hydrophobic interaction chromatography using an octyl-sepharose column CL-4B (2.5 cm x 10 cm) was performed. The column was washed with 200 ml of a buffer including 15% n-propanol and a 0.1 M sodium acetate buffer (pH 4.7), and LTA was collected using 300 ml of an elution buffer (35% n-propanol, 0.1 M sodium acetate, pH 4.7). The fragment including LTA was loaded into the column after adding an equilibration buffer (30% n-propanol, 0.1 M acetate buffer, pH 4.7) to re-perform DEAE-sepharose ion-exchange chromatography (1.5 cm x 10 cm). LTA was collected using 300 ml of an equilibration buffer including a series of NaCl (0 to 1 M).

Separation of LTA-derived glycolipid was performed by dissolving 30 mg of intact LTA in 500 ml of a 98% (v/v) aqueous acetic acid solution, sonicating the intact LTA for 30 minutes and heating the sonicated LTA at 100 °C for 3 hours. A solvent was removed under vacuum, and the glycolipid was then separated using chloroform/methanol/water (1:1:0.9, v/v/v). The glycolipid was extracted into an organic layer, and poly(glycerophosphate) was extracted into a solvent layer.

### Nuclear magnetic resonance (NMR) spectroscopy

An NMR experiment was performed using Avance-600 MHz and Avance-800 MHz high-resolution NMR spectroscopes equipped with a x,y,z-shielded gradient triple resonance probe or a z-shielded gradient triple resonance cryo probe. 10 mg of LTA was dissolved in 0.5 ml of ²H₂O solution. ²H₂O was used as a reference signal (4.75 ppm, ¹H). Homonuclear assignment was obtained from the spectra of correlation spectroscopy (COSY), double-quantum filtered correlation spectroscopy (DQF-COSY), total correlation spectroscopy (TOCSY), and rotating frame Overhauser enhancement spectroscopy (ROSEY). ¹³C assignment was based on the heteronuclear multiple-quantum correlation (HMQC) and heteronuclear multiple-bond correlation (HMBC) experiments. All the spectra were recorded at 300 K, and the NMR data was processed using the program nmrPipe28, and visualized using Sparky.

### Mild alkaline hydrolysis of glycolipid

In order to determine mild alkali-labile components of the glycolipid, 50 µg of a sample was dissolved in 200 µl of 0.5 M NaOH at 56 °C for 60 minutes. Thereafter, the resulting mixture was acidified with 3 N HCl, and then mixed with hexane. A supernatant including fatty acid was removed, and a lower phase solution was dried to remove salts. The residue was fractionated using a chloroform/methanol/water (8:4:3, v/v) solution and centrifuged to separate layers, and a supernatant was removed. A lower phase solution was dried again to remove salts. Then, the residue was dissolved in a chloroform/methanol (1:1, v/v) solution, and used for MALDI-TOF MS assay.

### Exoglycosidase treatment

Glycolipid was suspended in 50 µl of a 50 mM NH₄HC0₃ buffer containing 0.1% sodium tarurodeoxycholate, and α-glucosidase (*Saccharomyces cerevisiae*), β-glucosidase (almond), α-galactosidase (green coffee bean) or β-galactosidase (*E. coli*) was added, and treated at 37 °C for 48 hours. Thereafter, 1.35 ml of chloroform:methanol (1:1, v/v) and 100 µl of distilled water were added to separate layers, and a lower layer was dried and subjected to linkage analysis.

### MALDI-TOF MS

0.5 µl of a glycolipid solution was added to a matrix solution (2,5-dihydroxybenzoic acid solution, 30 mg/ml in 70% acetonitrile/30% water [v/v], 0.5 µl), and applied to a MALDI probe. The purified glycolipid was directly analyzed through IVIALDI-TOF MS, or analyzed after O-acetylation. The purified glycolipid was O-acetylated with 200 µl of a pyridine:acetic anhydride (1:1, v/v) at 80 °C for 2 hours. Thereafter, the solvent was removed, and the glycolipid was analyzed through MALDI-TOF MS.

### Partial hydrolysis of LTA with hydrofluoric acid

In order to achieve the partial or complete hydrolysis of a poly(glycerophosphate) backbone, 1 mg of LTA was added to 100 µl of 48% hydrofluoric acid (HF), and then treated at 4 °C for 5 to 48 hours. Thereafter, the resulting mixture was neutralized with saturated lithium hydroxide (LiOH), and then centrifuged (12,000 x g, 10 min) to remove a precipitate. A supernatant was collected and used for TLC and ESI-LIT MS analysis.

### Gas chromatography/mass spectrometry (GC/MS)

In order to analyze the monosaccharide compositions from an LTA hydrolysate, GC/MS analysis was performed. An authentic reference compound (galactose, glucose, mannose or N-acetylglucosamine) or an LTA hydrolysate sample was dissolved in pyridine, and then kept at room temperature for 48 hours. 50 µl of trimethylsilylimidazole was added, and the resulting mixture was heated at 67 °C for 30 minutes. The sample was dissolved in 200 µl of chloroform, and then subjected to a Finnigan MAT system (gas chromatography model GCQ, HP19091J-433). The sample was loaded into a nonpolar capillary column (5% phenyl methyl siloxane capillary 30 m x 250 µm i.d., film thickness of 0.25 µm, HP-5). The column was kept at 100 °C for 2 minutes, heated to 220 °C at a rate of 4 °C/minute, and kept for 5 minutes. Thereafter, the column was heated to 300 °C at a rate of 15 °C/minute, and kept for 5 minutes. A mass spectrometer was used in a positive ion chemical ionization mode having an ion sample temperature of 85 °C, an electronic energy of 65 eV and an emission current of 300 mA. Methane was used as an analyte gas, and its pressure was maintained at 0.5 Torr.

The fatty acid composition of the glycolipid were determined by adding 0.1 µg of tridecanoic acid to 200 µg of an LTA sample and hydrolyzing the LTA sample with 4 N KOH (100 °C, 4 hours). pH of a hydrolysate was adjusted to be close to 1.0, and free fatty acid was then extracted from chloroform (3 times, 1 ml), and dried with N₂ gas. The dried fatty acid was dissolved in acetonitrile (30 µl), and then treated with 10 µl of 35% pentafluorobenzyl bromide (in acetonitrile) and 10 µl of diisopropylethylamine. The resulting solution was heated at 40 °C for 20 minutes, and then dried with N₂ gas. The resultant pentafluorobenzyl ester was treated overnight with N,O-bis(trimethylsilyl)-trifluoroacetyl amide. A column was maintained at 85 °C, heated to 265 °C at a rate of 7 °C/minute, and then kept for 10 minutes. An amount of hydroxy fatty acid was determined using a selective ion detection method of detecting base peak ions, and the loss of an extract was corrected using an internal standard method using tridecanoic acid.

### Thin layer chromatography (TLC)

The analysis of the glycolipid was performed on a pre-coated silica gel HPTLC plate using chloroform-methanol-acetic acid-water (100:20:12:5, v/v/v/v). The glycolipid on TLC was detected when the glycolipid was treated with 5% sulfuric acid and heated at 120°C.

In order to analyze glycerol phosphate, TLC was performed using n-butanol-pyridine-water (15:30:20, v/v/v). 0.5% Ninhydrin (in butanol) and 5% sulfuric acid (in methanol) were used to detect alanine and a sugar substituent on TLC, respectively. Thereafter, the glycerol phosphate was heated at 120 °C to be visualized. Spots visualized on the TLC plate were collected, and a sample was extracted from a silica gel powder using methanol, and used for MS analysis.

### ESI-LIT MS

A mass spectrometer, Ultimate 3000 nano LC coupled to a LTQ-Orbitrap hybride linear ion trap, was used to perform liquid chromatography-tandem mass spectrometry. 5 µl of a sample was loaded, and Ultimate 3000 nano LC was then loaded into a Zorbax 300 Extended-C18 column (3.5 µm, 0.3 mm i.d x 150 length). Mobile phase A was 100% water including 0.1% formic acid, and mobile phase B was 100% acetonitrile including 0.1 % formic acid. Phase separation was performed using a C18 column. A sample separated from an LC system was analyzed using an electrospray ionization (ESI) technique. A sample solution was injected into an ion source through a metal capillary tube at a rate of 3 µl/minute and a high pressure (5 kV for glycerol phosphate and 4.5 kV for glycolipid). A capillary tube was maintained at 180 °C, and nitrogen was used as a sheath gas (at a flow rate of 8 arbitrary units). An analyzer was run in a negative ion mode. An ion entrance capillary voltage and a tube lens offset were -45 V and -206 V, respectively. A normalized collision energy of 35 to 37% and an isolation width of 2.5 to 3 Da were used for MSn fragmentation. The maximum ion collection time was set to 50 milliseconds, and an average value was calculated using three micro scans.

### Example 1: Structural analysis of LTA and LTA glycolipids

### Elucidation of entire structure of pLTA using ¹H, ¹³C and 2D-NMR spectra

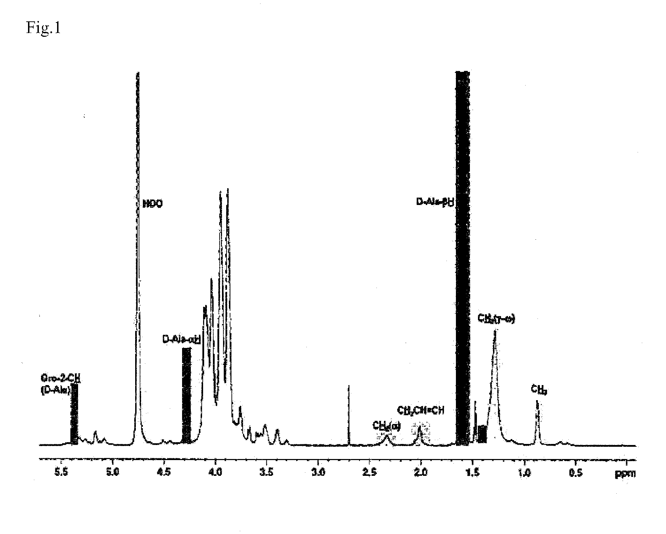

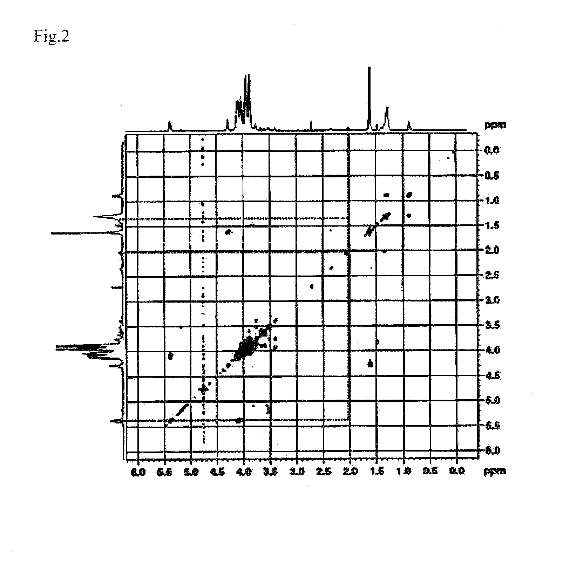

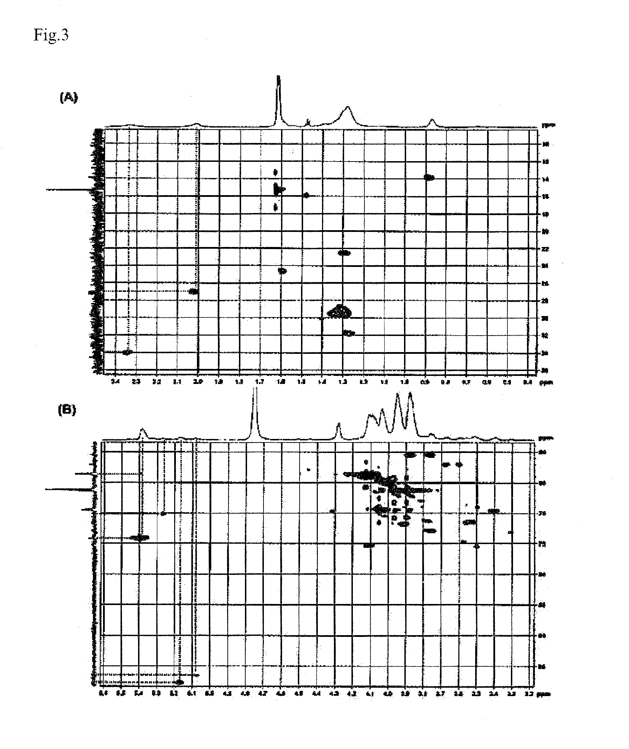

In the ¹H-NMR spectrum of aLTA, a resonance signal of an acetyl group of α-D-N-acetylglucosamine (GlcNAc) was reported to have a chemical shift (δ_{H}) of 2.1. In the ¹H-NMR spectrum of pLTA, however, a peak pattern at a chemical shift (δ_{H}) of 2.1 was different from that of aLTA. A resonance pattern of pLTA was distributed to a wider extent than that of aLTA, and a signal from a free acetyl group (δ_{H} 1.9) was not observed (FIG. 1). From the analysis results using the COSY spectrum, it was confirmed that δ_{H} 2.01 was associated with (γ-ω)-methylene- (-CH₂-; δ_{H} 1.28) and an olefin proton (-CH=CH-; δ_{H} 5.38) (FIG. 2). From the analysis results of the HMQC spectrum, it was also confirmed that protons at δ_{H} 2.01 were allotted from methylene (CH₂; δc 27) (FIG. 3A). Based on the analyses of the COSY and HMQC spectra, it was seen that a chemical shift (δ_{H}) of 2.01 was a signal from methylene attached to an alkenyl carbon (-CH₂-C**H**₂-CH=CH-C**H**₂-CH₂-) present on a fatty acid. As a result, it could be seen that pLTA had no GlcNAc substituent in the glycerol phosphate backbone.

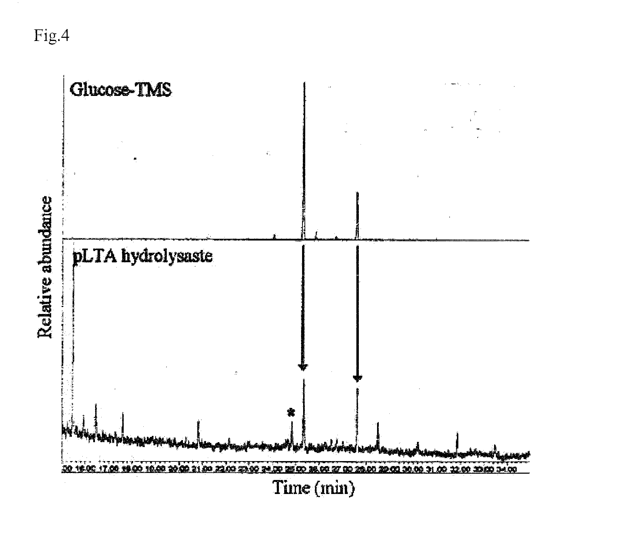

The presence of unsaturated fatty acid was also elucidated through interrelation of signals from δ_{H} 2.01 (C**H**₂-CH=CH) and δ_{H} 5.38 (C**H**=C**H**) (FIG. 2). From the fact that there were two anomeric proton signals [δH 5.08 (d, J=Hz) and 5.17 (d, J=3 Hz)] and two anomeric carbon signals (δc 96.5 and 97.7 ppm) in the ¹H and ¹³C NMR spectra, it was also confirmed that two sugar units were present in the glycolipid. Sugar measurement was performed through GC/MS analysis of an LTA hydrolysate (FIG. 4), and it was confirmed that the pLTA-derived glycolipid was substituted with a sugar such as glucose and galactose. Finally, it was confirmed that a J value of an anomer having two sugar units indicates that the anomeric center of D-pyranose was present in an α direction, signals from δ_{H} 5.08 and 5.17 were generated from anomeric protons of α-D-glucose and α-D-galactose, which correspond to the anomeric carbons resonated at δc 96.5 and 97.7, respectively (FIG. 3B). From these facts, it could be seen that hexoses of the pLTA-derived glycolipid were linked by means of an α-bond.

### Structural analysis of pLTA-derived glycolipid

A glycolipid sample of LTA was analyzed using an MALDI-TOF MS method.

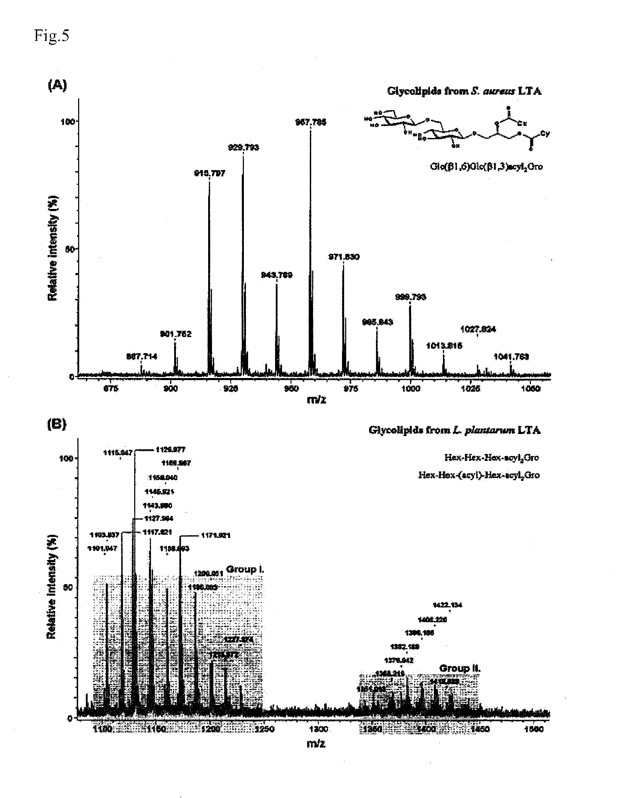

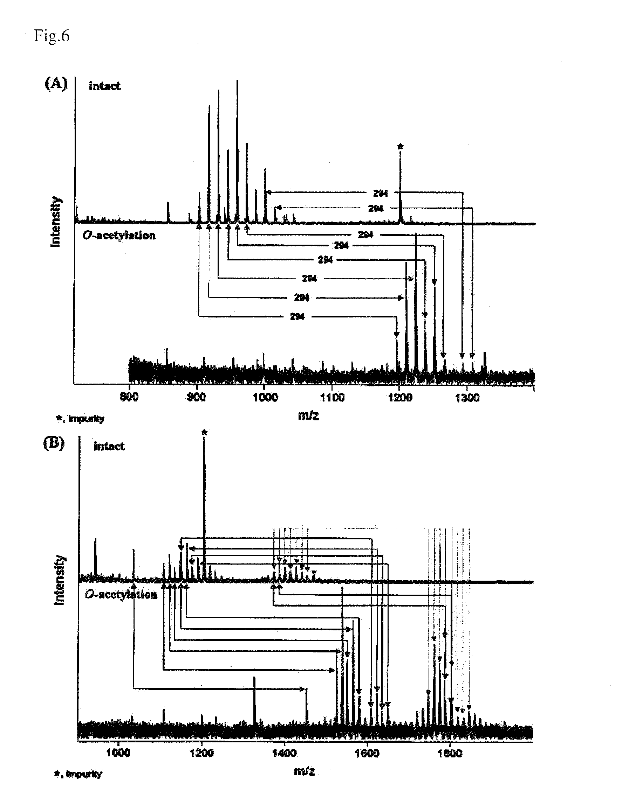

The mass spectrum of the glycolipid of pLTA was divided into two groups (FIG. 5B). An ion signal of Group I was confirmed to be in a range of 1,103 m/z to 1,227 m/z, and an ion signal of Group II was confirmed to be in a range of 1,368 m/z to 1,464 m/z. Glycolipids belonging to Group I were proved to be trihexosyl-diacyl-glycerol (Hex₃-DAG) and have a C₂₂ acyl chain at a C₁₆ position. Glycolipids belonging to Group II were proved to have an additional hexose residue or acyl chain. The additional hexose residue was confirmed by *O*-acetylation of the glycolipid (FIG. 6A).

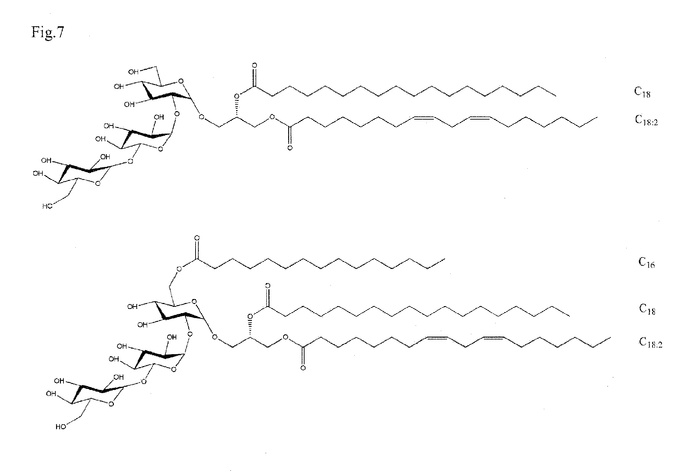

Acetylation of a hydroxyl residue causes an increase in mass unit of 42. The Group I glycolipid of pLTA showed an increased mass unit of 420 (10 hydroxyl residues), and was proved to be a trihexose group (FIG. 6B). Also, most of the glycolipids in Group II showed an increased mass unit of 378 (9 hydroxyl residues). According to the *O*-acetylation analysis, it could be seen that the glycolipids in Group II had an additional acyl chain at the hexose residue (FIG. 7).

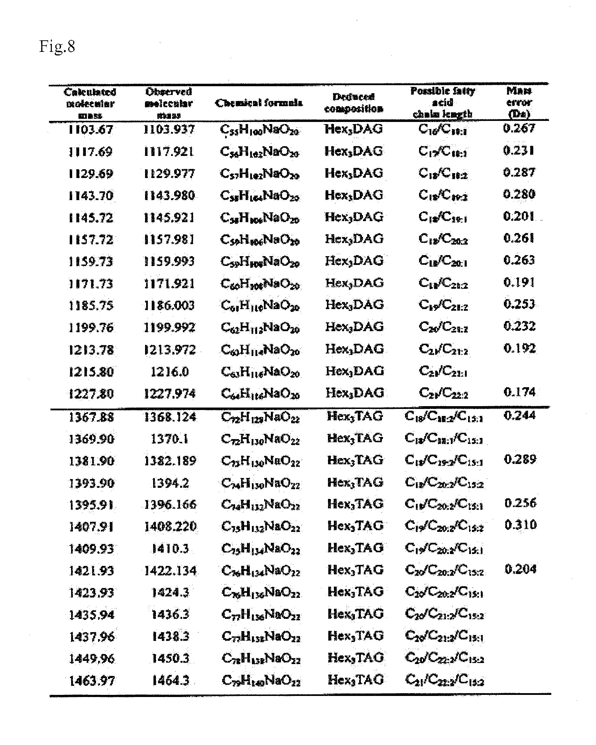

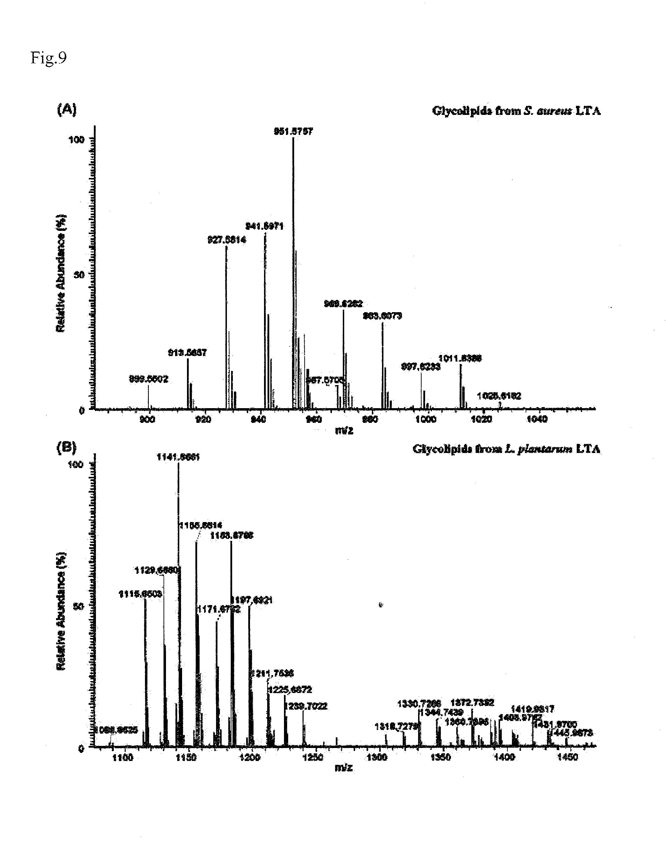

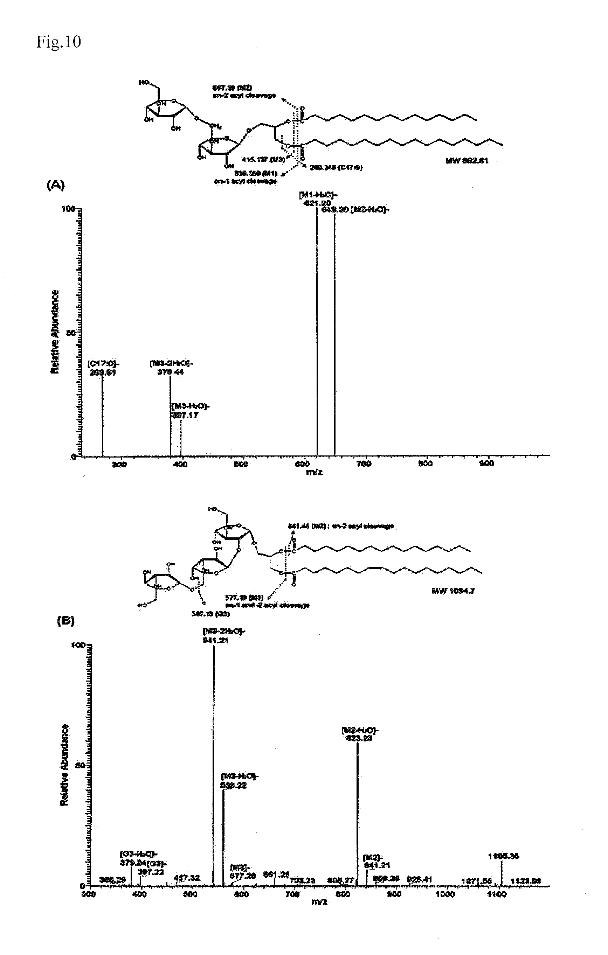

In addition, there was a difference in molecular mass of 12 Da in the spectrum, which indicates the addition or removal of an alkenyl carbon. From these facts, it was confirmed that the glycolipid had an unsaturated fatty acid chain. This probability was confirmed using COSY NMR (FIG. 2). Expected molecular components of the pLTA glycolipid are shown in FIG. 8. In order to perform the tandem MS analysis, the glycolipid was then analyzed using ESI-FT MS (negative ion mode) (FIG. 9), and a representative peak was selected from the ESI-FT MS results, and analyzed to determine the acyl chain distribution (FIGS. 10A and B).

As a result, it could be seen that the structure of the pLTA-derived glycolipid had three hexose backbones, each backbone was composed of trihexosyl-diacylglycerol (Group I) including an unsaturated fatty acid and trihexosyl-triacylglycerol (Group II), and the acyl chains had on average 18 carbon atoms, as shown in FIG. 9.

### Structural analysis of aLTA-derived glycolipid

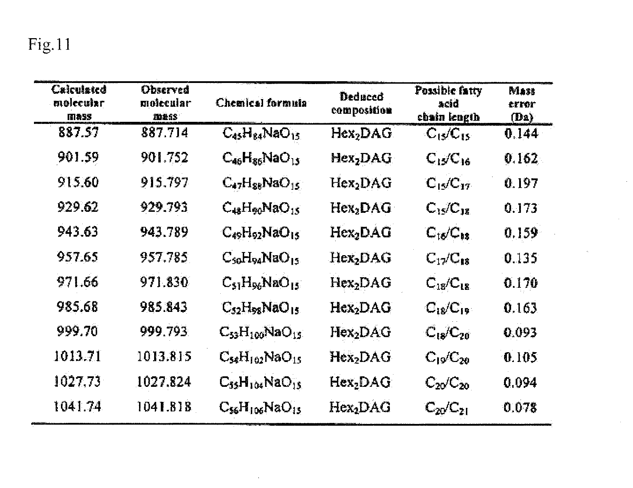

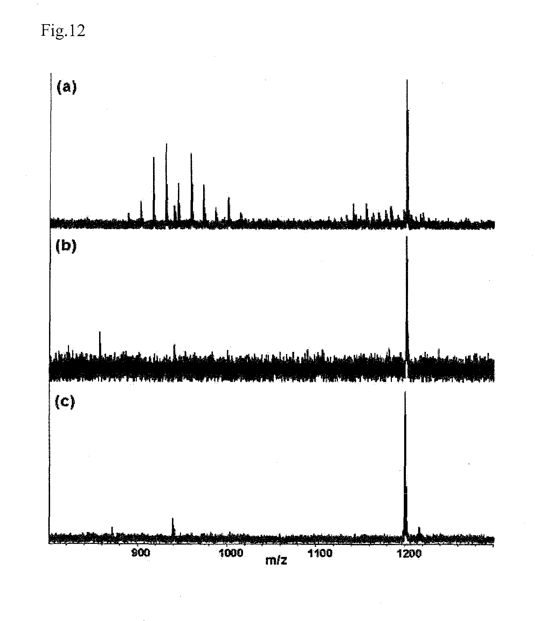

In the case of aLTA, it was confirmed that a ratio of electric charges (m/z) to a molecular mass of the glycolipid corresponded to a fatty acid having a C₁₅ to C₂₁ chain length (FIGS. 5A and 11). Almost all of the m/z signals in the glycolipid of aLTA were increased by a mass unit of 294. This indicates that the acetylation of 7 hydroxyl groups takes place and corresponds to two hexose residues. As a result, the glycolipid of aLTA was proven to have a dihexosyl group. Also, the presence of a fatty acid acyl chain in the glycolipid was confirmed through the mild alkaline hydrolysis (FIG. 12).

### Structural analyses of glycolipids of dLTA, rLTA and sLTA

In order to analyze the structures of glycolipid moieties of dLTA, rLTA and sLTA, the MALDI-TOF analysis was performed. dLTA, rLTA and sLTA represent *L*. *delbrueckii-, L. rhamnosus-* and *L.* sakei-derived LTAs, respectively.

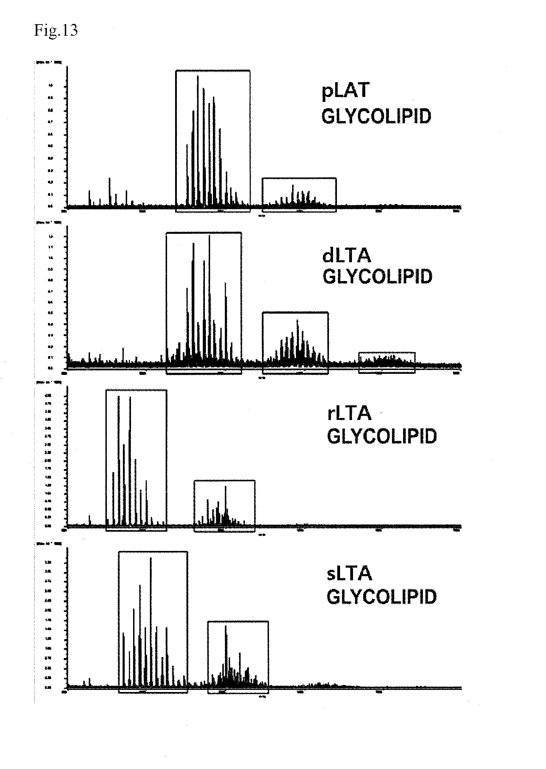

As shown in FIG. 13, the MALDI-TOF analysis results showed that the dLTA-derived glycolipid was divided into two groups, each of which has trihexosyl-diacylglycerol and trihexosyl-triacylglycerol, like the pLTA. In addition, a third group having four acyl chains was observed to be present in the dLTA-derived glycolipid. Also, each acyl chain was observed to have 18 average carbon atoms. The rLTA-derived glycolipid was generally observed to be divided into two groups, which had a lower molecular mass than the pLTA. Since one sugar had a molecular mass of 160 Da, it was confirmed that the dLTA-derived glycolipid had dihexosyl-diacylglycerol and dihexosyl-triacylglycerol, each of which was composed of two hexose backbones rather than three hexose backbones, and each acyl chain had 15 average carbon atoms. Also, it was confirmed that the sLTA-derived glycolipid had dihexosyl-diacylglycerol and dihexosyl-triacylglycerol, and had a longer acyl chain length than the rLTA since each acyl chain had on average 18 carbon atoms.

### Example 2: Anti-inflammatory effect of LTA-derived glycolipid

### Effect of LTA-derived glycolipid on suppression of TNF-α expression

THP-1 cells were treated with each of intact LTA, and an LTA-derived glycolipid and LTA-derived poly(glycerophosphate) separated from the intact LTA at a concentration of 100 µg/ml for 4 hours. Then, an expression level of TNF-α was measured from a culture solution using an ELISA method.

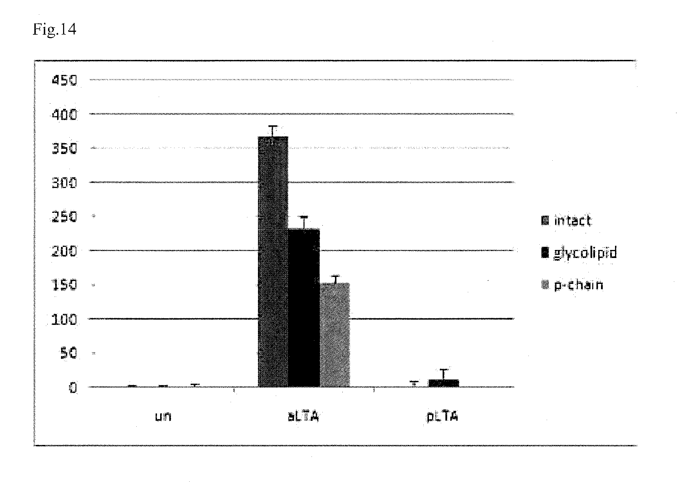

As shown in FIG. 14, the intact aLTA induced high expression of TNF-α, but the intact pLTA did not induce the expression of TNF-α. Also, the aLTA-derived glycolipid and poly(glycerophosphate) significantly induced the expression of TNF-α, which was lower than that of the intact aLTA, but the pLTA-derived glycolipid and poly(glycerophosphate) barely induced the expression of TNF-α. From these facts, it could be seen that the intact pLTA and the pLTA-derived glycolipid did not induce the expression of TNF-α unlike aLTA.

### Experiment on anti-inflammatorv effect by LTA modification

One experiment was performed to modify aLTA and pLTA and measure their activities. First, LTA was cultured overnight in Tris-HCl (pH 8.5) to remove D-alanine of the poly(glycerophosphate). Also, the LTA was treated with 0.1 N NaOH to remove a fatty acid acyl chain of the glycolipid. Thereafter, the resulting culture solution was neutralized, and modified LTA was purified using octyl chromatography, lyophilized, and quantified. Then, the modified LTA was used in this experiment. THP-1 cells were pre-treated with 100 µg/ml of D-alanine-free LTA (de-alanine) and fatty acid acyl chain-free LTA (de-acyl) for 18 hours, and then re-treated with 0.5 µg/ml of LPS for 4 hours. Then, an expression level of TNF-α was measured from the culture solution using an ELISA method.

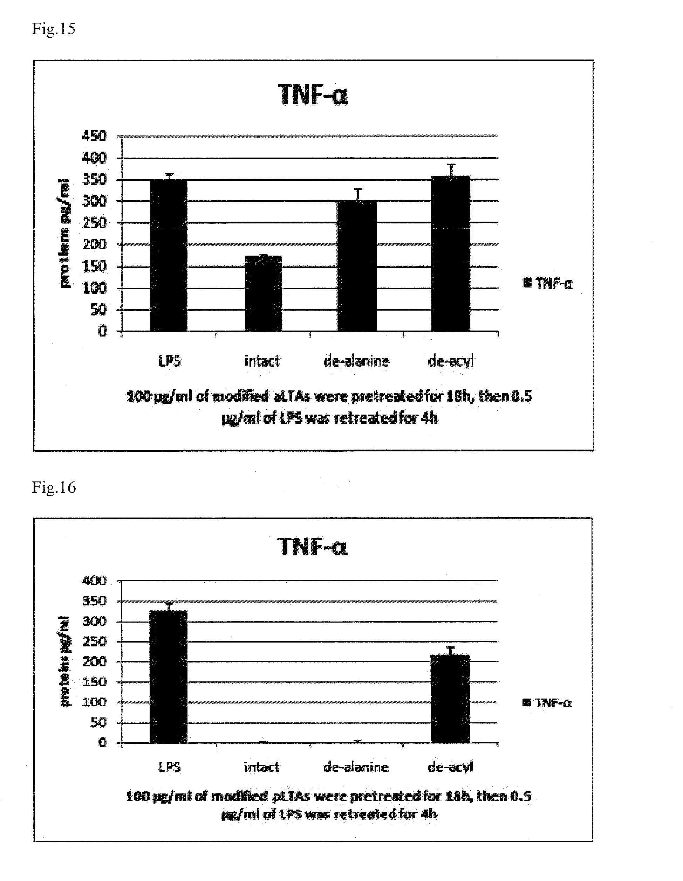

FIG. 15 shows the experimental results using aLTA. In the case of aLTA, slight suppression of TNF-α expression by LPS was observed in the THP-1 cell pre-treated with the intact aLTA, but the D-alanine-free and fatty acid acyl chain-free aLTAs (de-alanine, de-acyl) did not show this suppression effect.

FIG. 16 shows the results obtained from the same experiment using pLTA. Unlike the aLTA, the intact pLTA mostly suppressed the expression of TNF-α induced by LPS, and the pLTA (de-alanine) from which alanine of the poly(glycerophosphate) was removed showed the same effect. On the other hand, the pLTA (de-acyl) from which an acyl chain of the glycolipid was removed did not have an effect of suppressing the expression of TNF-α.

These results show that aLTA and pLTA have different biological characteristics. In particular, they show that the acyl chain of the glycolipid plays a more important role in the effect of pLTA on suppression of TNF-α expression, compared to the D-alanine in the poly(glycerophosphate).

### Anti-inflammatorv effect of pLTA-derived glycolipid

In order to examine an anti-inflammatory effect of a glycolipid separated from pLTA, THP-1 cells were pre-treated with various concentrations of the glycolipid for 20 hours, and then re-treated with flexPGN at a concentration of 10 µg/ml for 4 hours. Thereafter, an expression level of TNF-α was measured from a culture solution using an ELISA method.

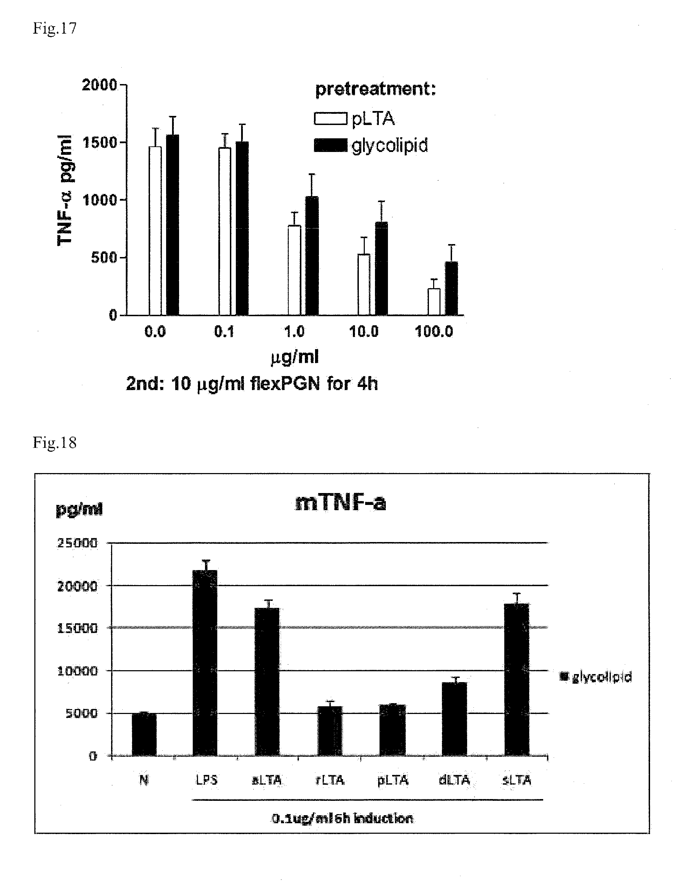

As shown in FIG. 17, when the THP-1 cells were treated with 10 µg/ml of flexPGN only, the expression of TNF-α was highly induced. However, when the THP-1 cells were pre-treated with the glycolipid, it was seen that the expression of TNF-α was decreased according to the concentration of the pre-treated glycolipid. Such an expression suppression effect was less excellent compared to the same concentration of the intact pLTA, but these results show that only the glycolipid moiety of pLTA has a sufficient effect of suppressing the expression of TNF-α.

### Anti-inflammatory effects of other LTA-derived glycolipids

THP-1 cells (5×10⁵ cells/ml) were treated at a concentration of 0.1 µg/ml for 6 hours with LPS (LPS from *E. coli* 111:B4) and a glycolipid separated from each LTA (aLTA, rLTA, pLTA, dLTA or sLTA) using the method as described in Experiment Examples, respectively. Thereafter, an expression level of TNF-α was measured from a culture solution using an ELSIA method.

FIG. 18 shows the effects of glycolipids isolated from different LTAs of suppressing expression of TNF-α. It could be seen that the aLTA- and sLTA-derived glycolipids hardly had the effect of suppressing the expression of TNF-α, but the rLTA-, pLTA- and dLTA-derived glycolipids very effectively suppressed the expression of TNF-α.

### Example 3: In vitro experiment on anti-inflammatory effect of pLTA

### Experiment on effects of pLTA on suppression of expression of Shigella flexneri peptidoglycan (PGN)-induced TNF-α and IL-1β

Human monocytes, THP-1 cells, were cultured under the conditions of 37 °C and 5% CO₂ in an RPMI 1640 medium supplemented with heat-treated 10% inactive FBS, 100 U/ml penicillin and 100 µg/ml streptomycin. In order to perform cytokine induction using the THP-1 cells, the THP-1 cells were transferred to a 96-well plate at a density of 4 x 10⁵ cells/well, and incubated for 24 hours under the conditions of 37 °C and 5% CO₂ to stabilize the cells. Then, the THP-1 cells were pre-treated with various concentrations (0 to 100 µg/ml) of *p*LTA for 24 hours, and then re-treated with peptidoglycan at a concentration of 10 µg/ml for 4 hours.

Measurement of expression levels of TNF-α and IL-1β was performed using a conventional sandwich ELISA method after a cell culture was centrifuged to collect a cell supernatant. ELISA experiments were performed, respectively, using anti-TNF-α monoclonal mouse IgG1 (clone #28410)/biotinylated anti-human TNF-α specific goat IgG and anti-human IL-1β monoclonal mouse IgG1 (clone 2805)/biotinylated anti-human IL-1βspecific goat IgG, which were commercially available from R & D systems. After treatment with streptavidin HRP, *O*-phenylenediamine was used as a substrate to emit light. A value obtained by subtracting an OD value measured at 450 nm from an OD value measured at 550 nm on an ELISA reader was used as an effective value.

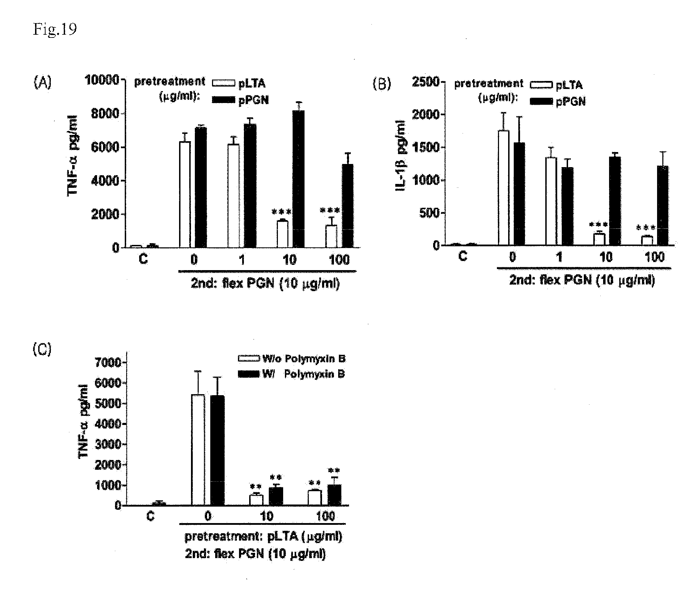

As shown in FIG. 19, when the THP-1 cells were pre-treated with LTA, it was revealed that the expression of TNF-α (FIG. 19A) and IL-1β (FIG. 19B) whose expression was increased by peptidoglycan was decreased with an increasing concentration of LTA. In order to determine whether or not the expression of inflammatory cytokines is induced by an endotoxin, experiments using polymyxin B were performed. Polymyxin B functions to bind to lipid A of LPS and suppress the activity of LPS. Therefore, if the experimental results were due to the endotoxin, the expression of the inflammatory cytokines was not suppressed when the THP-1 cells were treated with polymyxin B. However, the suppression of the TNF-α expression by pLTA was induced in a medium supplemented with polymyxin B, which indicates that the tolerance by LTA is not induced by endotoxin (LPS) (FIG. 19C).

Peptidoglycan from gram-negative bacteria functions to enhance expression of a large amount of pro-inflammatory cytokines from immunocytes by stimulating NOD2 in the immunocytes. However, specific expression of the pro-inflammatory cytokines by peptidoglycan of the gram-negative bacteria may be suppressed by pre-treating the immunocytes with pLTA.

### Experiment on effects of PLTA on suppression of expression of peptidoglycan-induced NOD2

Changes in expression of a septicemia-associated receptor, NOD2, by pLTA were measured as follows. THP-1 cells were transferred to a 24-well plate at a density of 8 x 10⁵ cells/well, and incubated for 24 hours under the conditions of 37 °C and 5% CO₂ to stabilize the cells. The THP-1 cells were pre-treated with pLTA at a density of 100 µg/ml for 24 hours, re-treated with *S. flexneri* peptidoglycan at a density of 10 µg/ml, and then incubated form 2 hours. A culture solution was centrifuged to collect cells. Then, cDNA was synthesized from a total of RNA, and subjected to real-time PCR to measure a change in expression of NOD2. A change in NOD2 protein level was measured using a western blotting method. The THP-1 cells pre-treated with pLTA under the above-described conditions were treated with peptidoglycan for 2 hours. The THP-1 cells were sonicated, and an SDS-PAGE loading dye was added thereto. Thereafter, the THP-1 cells were boiled for 5 minutes, and subjected to 12% SDS-PAGE to separate proteins. The separated proteins were transferred to a nylon membrane, and subjected to western blotting using anti-TLR2, anti-TLR4 and anti-NOD2 antibodies.

In order to elucidate the role of NOD2 in inducing an excessive inflammatory response by *S. flexneri* peptidoglycan, experiments were performed as follows. U937 cells were transformed with an NOD2 expression vector and a pNF-κB-Luc/pRL-SV40 vector, respectively. After 24 hours, the respective transformed cells were pre-treated with pLTA at a density of 100 µg/ml for 24 hours, and re-treated with peptidoglycan at a density of 10 µg/ml for 12 hours. A change in activity of NF-κB according to the NOD2 expression level was measured by dividing the activity of NF-κB measured using a microplate reader by the activity of *Renilla.*

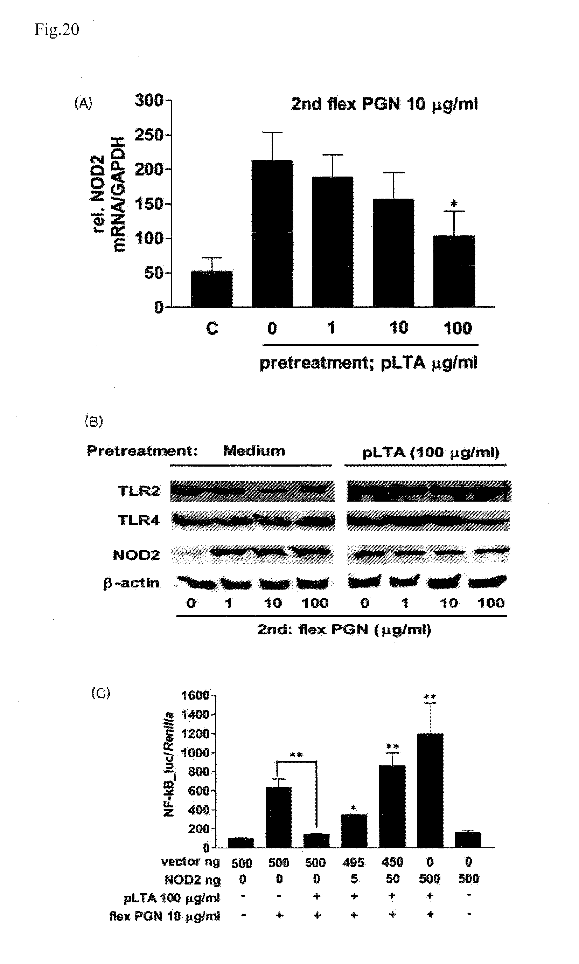

As shown in FIG. 20, the *S. flexneri* peptidoglycan remarkably increased the expression of NOD2 in the THP-1 cells. On the other hand, the expression of NOD2 mRNA in the THP-1 cells pre-treated with pLTA tended to decrease with an increasing concentration of the pre-treated pLTA (FIG. 20A), and an expression level of NOD2 protein in the THP-1 cells pre-treated with pLTA was also significantly decreased compared to the THP-1 cells that were not pre-treated with pLTA (FIG. 20B). These results indicate that the signal transmission is caused by interaction between the septicemia-associated receptor, NOD2, and the peptidoglycan since pLTA functions to suppress the expression of NOD20.

Also, it was confirmed that the activity of the peptidoglycan-induced NF-κB in the THP-1 cells pre-treated with pLTA was significantly decreased compared to the THP-1 cells that were not pre-treated with pLTA (FIG. 20C). However, when the THP-1 cells were transformed with an NOD2 expression vector, such suppression of NF-κB activity disappeared. These results indicate that NOD2 functions as a major receptor of peptidoglycan, and the activity of NF-κB which is a major transcriptional factor of NOD2 is also suppressed by pre-treating the THP-1 cells with pLTA, thereby inhibiting the mRNA expression of NOD2.

### Experiment on pLTA and TLR2 relevance

In order to examine relevance to TLR2 in a suppression action of expression of cytokines by pLTA, TLR2 siRNA experiments were performed. An siRNA duplex targeting TLR2 was purchased from Santa Cruz Biotechnology (Santa Cruz, USA).

THP-1 cells were transformed with TLR2 siRNA and the control siRNA using a TransIT-TKO infection reagent (Mirus, WI), and then incubated for 72 hours. The THP-1 cells transformed with the TLR2 siRNA and control siRNA were pre-treated with pLTA at a density of 100 µg/ml for 24 hours, and then re-treated with peptidoglycan at a density of 10 µg/ml for 4 hours. Thereafter, an expression level of TNF-α was measured from a culture solution using an ELISA method.

As another experiment, a bone marrow-derived macrophage (BMM) was isolated from a mouse (C57BL/6) in which TLR2 was knocked out, seeded into a 96 well plate, and incubated at 37 °C and 5% CO₂ for 24 hours. BMM was pre-treated with pLTA at a density of 100 µg/ml for 24 hours, and then re-treated with peptidoglycan at a density of 10 µg/ml for 4 hours. Thereafter, an expression level of TNF-α was measured from a culture solution using an ELISA method.

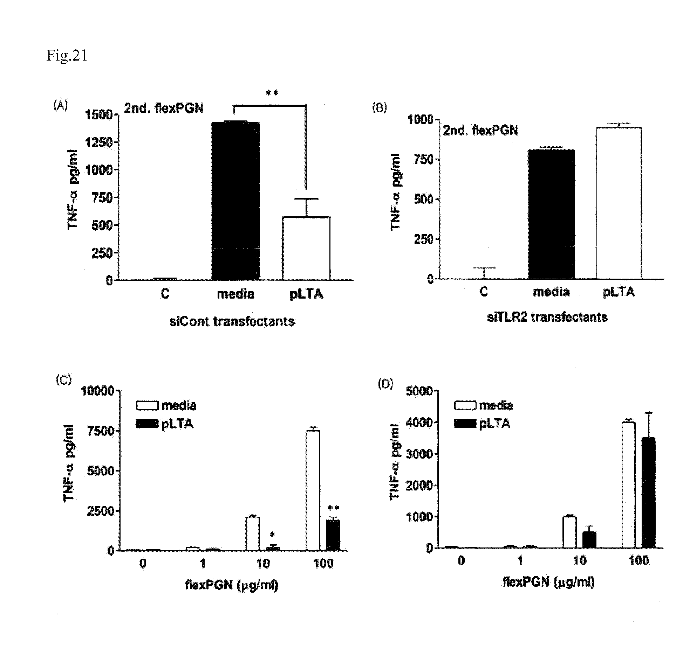

As shown in FIG. 21, the expression of the peptidoglycan-induced TNF-α was decreased since the tolerance induction by pre-treatment with pLTA was caused in the THP-1 cells transformed with the control siRNA (FIG. 21A). On the other hand, a decrease in such expression was not observed in the THP-1 cells transformed with TLR2 siRNA (FIG. 21B). These results were also observed in the BMM in which TLR2 was knock-out. In the case of BMM isolated from a normal mouse, the expression of TNF-α was increased according to the concentration of peptidoglycan, and such increased expression was decreased by pre-treatment with pLTA (FIG. 21C). On the other hand, in the case of the TLR2^{-/-} BMM, it was revealed that the expression of TNF-α was increased according to the concentration of peptidoglycan, regardless of the pre-treatment with pLTA (FIG. 21D). As a result, the tolerance induction of pLTA due to overexpression of the peptidoglycan-induced TNF-α indicates that TLR2 functions as a necessary receptor.

### Suppression of NF-κB activity and TNF-α expression by pLTA

Overexpression of TNF-α by treatment with endotoxin LPS was caused by the phosphorylation of MAP kinase and activity of NF-κB. In order to determine an effect of pLTA on the phosphorylation of MAP kinase and activity of NF-κB, western blotting and immunofluorescence staining experiments were performed as follows.

Western blotting: THP-1 cells were transferred to a 24-well plate at a density of 8 x 10⁵ cells/well, and then incubated for 24 hours under the conditions of 37 °C and 5% CO₂ to stabilize the cells. The THP-1 cells were pre-treated with pLTA at a density of 100 µg/ml for 24 hours, LPS was added at a concentration of 0.1 µg/ml, and the THP-1 cells were incubated for 1 additional hour. A culture solution was centrifuged to collect the cells, and the collected cells were sonicated in a *Laemmli* buffer. An SDS-PAGE loading dye was added to the lysed cells, and the resulting cells were boiled for 5 minutes, and then subjected to 12% SDS-PAGE to separate proteins. The separated proteins were transferred to a nylon membrane, and then western-blotted using an anti-phospho MAP kinase antibody (anti-phospho ERK and anti-phospho JNK), an anti-NF-κB antibody and an anti-β-actin antibody.

Immunofluorescence staining: THP-1 cells were transferred to a 24-well plate at a density of 8 x 10⁵ cells/well, and then incubated for 24 hours under the conditions of 37 °C and 5% CO₂ to stabilize the cells. The THP-1 cells were pre-treated with pLTA at a density of 100 µg/ml for 24 hours, re-treated with LPS at a concentration of 0.5 µg/ml, and then incubated for 2 hours. The incubated cells were centrifuged for 15 minutes at 3,500 rpm to be attached onto a slide glass, and then immobilized with 4% para-formaldehyde. Thereafter, the permeability through a cell membrane was enhanced with 0.5% Triton X-100, and the immobilized cells were treated with anti-NF-κB p50 and anti-NF-κB p65 antibodies for 2 hours. Then, the cells were treated with a donkey-derived anti-mouse IgG-FlTC antibody for 90 minutes, and phosphorylation levels of NF-κB p50 and NF-κB p65 in the cells were measured using a confocal microscope.

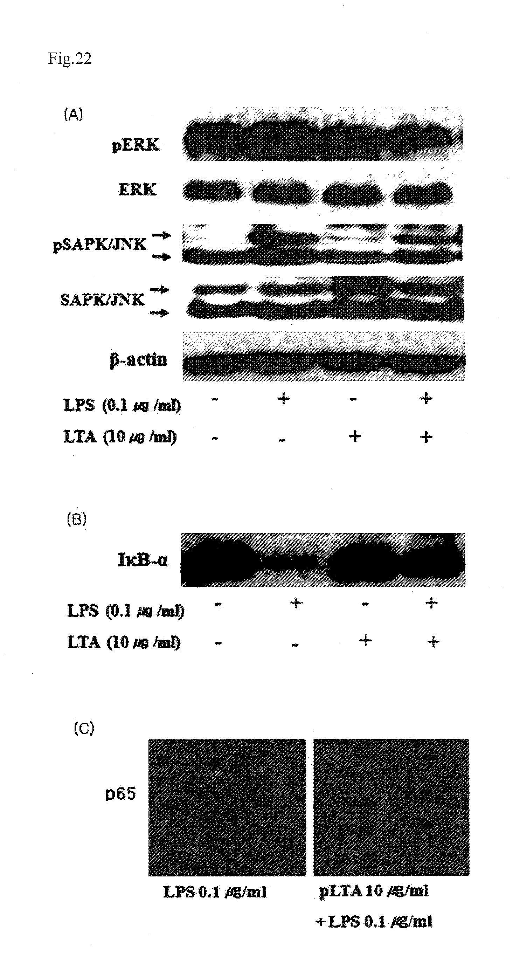

As shown in FIG. 22, it was revealed that the activity of MAP kinase was decreased by treatment with pLTA (FIG. 22A). That is, it was observed that the activities (phosphorylation) of ERK and JNK were suddenly increased 60 minutes after treatment of the THP-1 cells that did not undergo pLTA tolerance with LPS. On the other hand, it could be seen that the activities of ERK and JNK in the cells undergoing the pLTA tolerance were decreased, compared to the cells that did not undergo the pLTA tolerance.

The activity of the LPS-induced NF-κB requires phosphorylation of IKK kinase. The activated IKK kinase induces phosphorylation a group of I-κB proteins to separate I-κB from NF-κB. The NF-κB was translocated into the nucleus to facilitate cell attachment and transcription of genes such as cytokines and cytokine receptors. From these experimental results, it was revealed that degradation of I-κBα was accelerated after the treatment with LPS in the case of the cells that did not undergo pLTA tolerance. However, it was confirmed that I-κBα was poorly degraded in the case of the cells undergoing the pLTA tolerance (FIG. 22B). These results indicate that the activity of NF-κB in the cells undergoing the pLTA tolerance is suppressed, compared to the cells that did not undergo pLTA tolerance.

In order to determine these results, an immunofluorescence staining method using an anti-NF-κB antibody was performed to confirm that the NF-κB activity was suppressed by the pLTA tolerance (FIG. 22C). Such suppression of the activities of factors associated with a series of signal transmissions indicates that pLTA plays a major role in suppressing overexpression of TNF-α induced by LPS.

### Example 4: In vivo experiment on anti-inflammatory effect of pLTA

### Effect of pLTA on suppression of septicemia

pLTA or PBS was peritoneally injected into fifteen 4-week-old male mice (BALB/c) at a concentration of 300 mg/kg. After 24 hours, 200 µl of a septicemia-induced aqueous solution (250 mg/kg aLTA + 25 mg/kg MDP) was peritoneally injected to inflict an endotoxic shock on the mice. The viability of the mice was observed over 4 days after septicemia induction.

As another experiment, the five mice peritoneally injected with pLTA and the five mice peritoneally injected with PBS were injected with a septicemia-inducing substance. After 12 hours, blood was taken from the mice's hearts, a serum was isolated, and a content of TNF-α in the blood was then measured using an ELISA method.

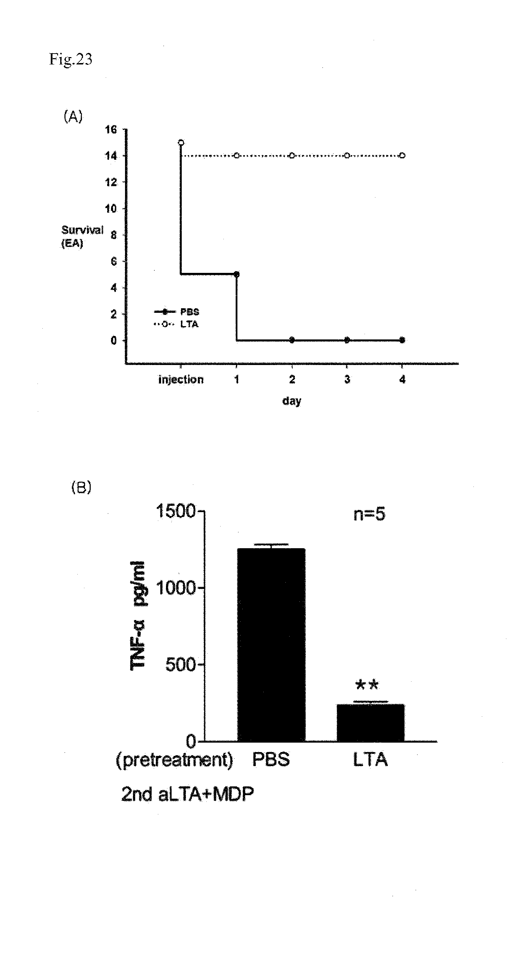

As shown in FIG. 23, all the mice pre-treated with PBS died within 48 hours when peritoneally injected with a septicemia inducing factor. On the other hand, the mice pre-treated with pLTA showed a viability of approximately 90% on day 4 (FIG. 23A). In the case of the mice pre-treated with pLTA, it was also confirmed that an amount of TNF-α found in the blood was measured to be significantly reduced, compared to that of TNF-α in the blood from the mice pre-treated with PBS (FIG. 23B).

These results indicate that pLTA is effective in suppressing the excessive inflammatory response induced *in vivo* by aLTA+MDP and also inhibiting gram-positive bacteria-derived septicemia.

### Alleviation of atopic skin disease by treatment with pLTA

An atopic skin disease breaks out due to a variety of causes such as bacterial infection, exposure to chemicals, etc. 4-week-old SPF (specific pathogen free) male ICR mice (Central Lab. Animal Inc.) and 4-week-old SPF male NC/Nga mice (Central Lab. Animal Inc.) were purchased, adapted in a laboratory animal breeding room for 7 days, and then used for experiments. The mice were divided into two experimental groups: two groups of 20 ICR mice and two groups of 5 NC/Nga mice. In all the experimental groups, hair was cut at dorsal regions of the mice the day before application of a test material, and atopic dermatitis was induced by applying 1% DNCB to the mice's dorsal regions twice at intervals of 4 days referring to the previous research, followed by applying a 0.2% DNCB solution to the mice's dorsal regions at intervals of 3 days over 4 weeks. After the outbreak of atopy, pLTA was peritoneally injected into the mice at a concentration of 350 µg/kg, and skin lesions were then observed. An immuno-histochemistry (IHC) method was used to measure the distribution and level of a cytokine (IL-4) associated with dermatitis and atopy in the skin tissue around the dermatitis pruritus in connection with the Th2 deviation in blood.

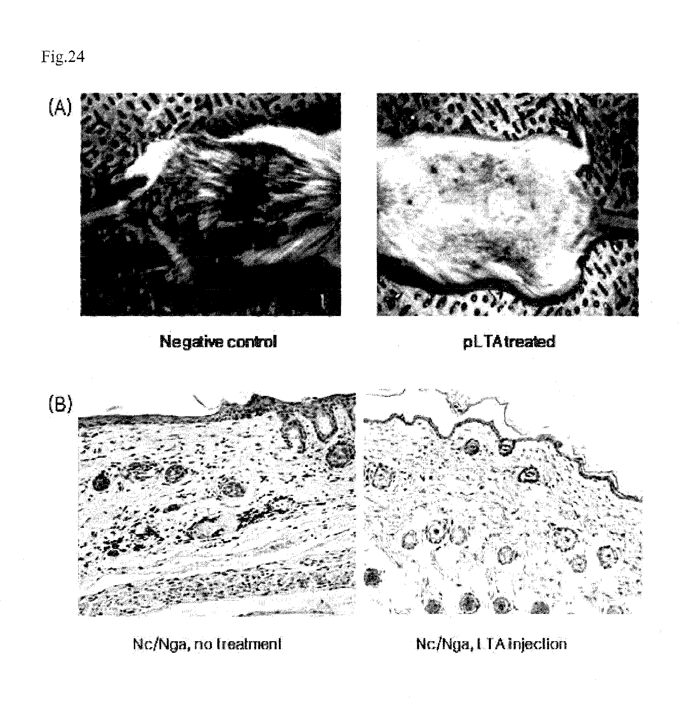

As shown in FIG. 24, when DNCB was applied to the mice's dorsal regions, the crust ecdysis and abscessed papule as well as erythema and edema over the dorsal regions were observed with the naked eye in both of the ICR mouse model and the NC/Nga mouse model, and severe symptoms such as bleeding were observed according to populations. From these results, it was revealed that such skin lesions were substantially similar to symptoms of human atopic dermatitis. Also, dermatitis was strongly induced compared to other mouse models. However, these symptoms were observed to be alleviated by injection of pLTA (FIG. 24A). In order to check the Th2 deviation that may be caused by atopic dermatitis, IHC was performed to measure a level of related cytokines in the affected skin tissue. In both of the ICR and NC/Nga mouse models, it was revealed that IL-4 was distributed at a significantly increased level in the skin tissue, but IFN-r was distributed at a similar level. On the other hand, in the case of the mice treated with pLTA, it was revealed that the distribution of IL-4 in the skin tissue was decreased (FIG. 24B), which indicates that pLTA functions to alleviate the symptoms of atopy by suppressing the expression of IL-4 that induces the Th2 response.

### Example 5: Experiment on anti-inflammatory effect of other LTAs

### Anti-inflammatory effect of rLTA

In order to examine an anti-inflammatory effect of LTA separated from another kind of the lactobacillus, *L. rhamnosus* (rLTA), THP-1 cells were treated with rLTA at a concentration of 100 µg/ml, and stimulated for 4 hours. Thereafter, an expression level of TNF-α was measured from a culture solution using an ELISA method.

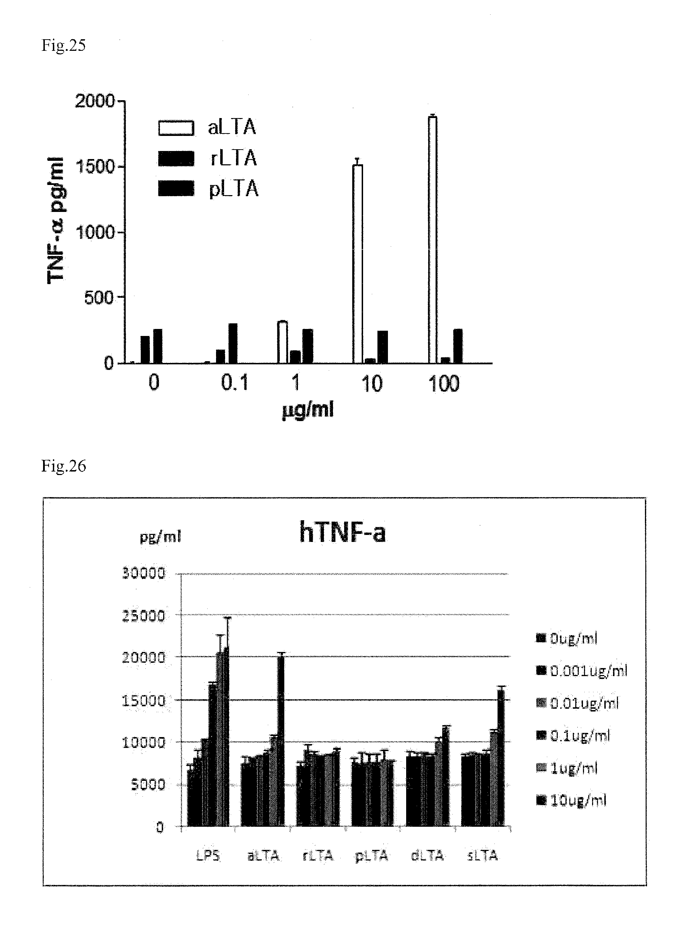

As shown in FIG. 25, as the THP-1 cells were pre-treated with an increasing concentration of rLTA, an expression level of TNF-α was reduced.

### Comparison of anti-inflammatory effects of aLTA, rLTA, pLTA, dLTA and sLTA

THP-1 cells were treated with LPS and each LTA to determine an expression level of TNF-α.

As shown in FIG. 26, when the THP-1 cells were treated with LPS and each LTA at a concentration of 10 µg/ml, it could be seen that aLTA and sLTA did not suppress the expression of TNF-α, but the other LTAs effectively suppressed the expression of TNF-α.

Also, it was assumed that having different expression patterns of TNF-α according to the kinds of LTAs is based on the fact that individual LTAs have different effects on the cell signal pathway due to their structural difference. In order to verify the assumption, THP-1 cells were treated with a signal inhibitor associated with the cell signal pathway, and production of TNF-α by LTA was confirmed.

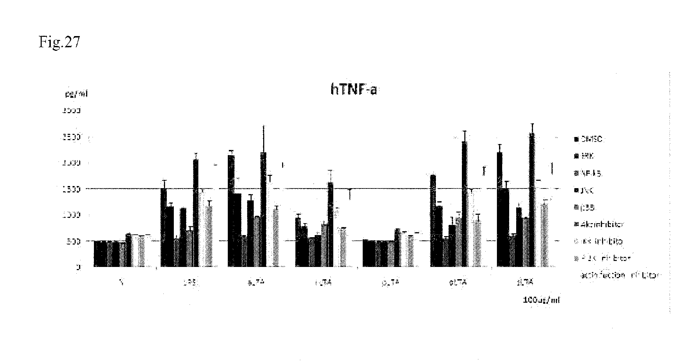

As a result, it was seen that the expression of TNF-α was decreased or increased when each LTA was treated with a certain signal inhibitor, as shown in FIG. 27 and listed in the following Table 1 in which the expression suppression rates of individual LTAs were quantified. From these facts, it was confirmed that the structural difference of LTA had different effects on certain cell signal pathways.

**Table 1**

| | TNF-a | DMSO | ERK | NF-kB | JNK | p38 | Akt | IKK | P13K | AFI |
|---|---|---|---|---|---|---|---|---|---|---|
| aLTA | 4,8 | -46,7 | -31.8 | -9.1 | -13.6 | -40.0 | -7.3 | -10,3 | 8.3 | -18.8 |
| pLTA | 64.3 | 66.7 | 54,5 | 9.1 | 54.5 | 28.6 | 63.4 | 51.7 | 50.0 | 56.3 |
| dLTA | 42.9 | -16.7 | -9.1 | 0.0 | 27.3 | -28.6 | -17.1 | 0.0 | 25.0 | -9.4 |
| rLTA | 57.1 | 36.7 | 31.8 | 0.0 | 45.5 | -14.3 | 22.0 | 27.6 | 37.5 | 18.8 |
| sLTA | 23,8 | -46.7 | -36.4 | -9.1 | 0.0 | -28.6 | -26.8 | -3.4 | -4.2 | -9.4 |

### Example 6: Induction of antibody response using pLTA-derived glycolipid

A mouse was primarily immunized with 2.5 µg of hepatitis B virus surface antigen ("HBsAg") on day 0. Then, the mouse was secondarily immunized on day 21 (200 µl/injection). Blood was taken from the mouse 20 and 27 days after the primary and secondary immunizations. Serum was collected, and subjected to standard ELISA using an anti-HBsAg antibody. The following Table 2 shows that the glycolipid according to the present invention induces production of an anti-HBsAg antibody when administered to an animal in combination with HBsAg.

**Table 2**

| | Anti-HBsAg titer⁻¹ | | | |
|---|---|---|---|---|
| | IgG₁-specificity | | IgG₂ₐ-specificity | |
| | 20 days after primary immunization | 27 days after secondary immunization | 20 days after primary immunization | 27 days after secondary immunization |
| pLTA glycolipid (30 µg) | 21,000 | 360,000 | 43,000 | 540,000 |
| pLTA glycolipid (10 µg) | 6000 | 190,000 | 10,000 | 250,000 |
| Vehicle | 3,000 | 90,000 | 2,000 | 70,000 |
| PBS | 1,000 | 20,000 | 1,000 | 25,000 |

### Preparative Example 1: Medicine including pLTA

### Preparation of powder

10 mg ofpLTA or pLTA analogueE
100 mg of lactose
10 mg of talc

The above-described components were mixed, and then filled in an airtight package to prepare a powder.

### Preparation of tablet

5 mg of pLTA or pLTA analogue
100 mg of corn starch
100 mg of lactose
2 mg of magnesium stearate

The above-described components were mixed, and then pressed according a conventional method of preparing a tablet to prepare a tablet.

### Preparation of capsule

10 mg of pLTA or pLTA analogue
3 mg of crystalline cellulose
14.8 mg of lactose
0.2 mg of magnesium stearate

The above-described components were mixed, and then filled in a gelatin capsule according a conventional method of preparing a capsule to prepare a capsule.

### Preparation of injection

50 mg of pLTA or pLTA analogue
180 mg of mannitol
2,974 mg of sterile distilled water for injection
26 mg of Na₂HPO₄•12H₂O

An injection was prepared according to a conventional method of preparing an injection so that the above-described contents of the components were contained per ampoule (2 ml).

### Preparation of solution

10 mg of pLTA or pLTA analogue
10 g of isomerized sugar
5 g of mannitol
Suitable amount of distilled water

A solution was prepared according to a conventional method of preparing a solution. That is, the above-described components were added and dissolved in distilled water, and a suitable amount of lemon flavor was added. Thereafter, the components were mixed, and distilled water was added to a total amount of 100 ml. The resulting mixture was filled in a brown bottle and sterilized to prepare a solution.

### Preparative Example 2: Heath food including pLTA

100 mg ofpLTA or pLTA analogue
Suitable amount of vitamin mixture
70 µg of vitamin A acetate
1.0 mg of vitamin E
0.13 mg of vitamin B₁
0.15 mg of vitamin B₂
0.5 mg of vitamin B₆
0.2 µg of vitamin B₁₂
10 mg of vitamin C
10 µg of biotin
1.7 mg of nicotinamide
50 µg of folic acid
0.5 mg of calcium pantothenate
Suitable amount of inorganic matter mixture
1.75 mg of ferrous sulfate
0.82 mg of zinc oxide
25.3 mg of magnesium carbonate
15 mg of potassium phosphate monobasic
55 mg of potassium phosphate dibasic
90 mg of potassium citrate
100 mg of potassium carbonate
24.8 mg of magnesium chloride

The components relatively suitable for health foods were mixed to adjust a composition ratio of the vitamin and mineral mixture in preferred Examples, but the blending ratio may be optionally varied. The components were mixed, and a granule was prepared according to a conventional method of preparing a health food, and the granule may be used to prepare a health food composition according to the conventional method.

### Preparative Example 3: Beverage including pLTA

100 mg of pLTA or pLTA analogue
15 g of vitamin C
100 g of vitamin E (powder)
19.75 g of ferrous lactate
3.5 g of zinc oxide
3.5 g of nicotinamide
0.2 g of vitamin A
0.25 g of vitamin B₁
0.3g of vitamin B₂
Quantitative amount of water

A beverage was prepared according to a conventional method of preparing a health beverage. That is, the above-described components were mixed, and then heated at 85 °C for approximately 1 hour while stirring. Thereafter, the resulting solution was filtered and put into a sterile 2 L container, which was sealed, sterilized and stored in a refrigerator. Finally, the solution was used to prepare a health beverage composition according to the present invention.

The components relatively suitable for fancy beverages were mixed to adjust a composition ratio of the components in preferred Examples, but the blending ratio may be optionally varied according to the regional or national preference of a consumer class or a consumer nation and to applications.

### Preparative Example 4: Beverage including pLTA

### Preparation of skin-softening lotion

0.1 % by weight of pLTA or pLTA analogue
3.0% by weight of glycerin
2.0% by weight of butylene glycol
2.0% by weight of propylene glycol
1.00% by weight of polyoxyethylene (60) hydrogenated castor oil
10.0% by weight of ethanol
0.1 % by weight of triethanolamine
Trace amount of antiseptic
Trace amount of pigment
Trace amount of spice
Balance of distilled water

### Preparation of nutritional skin lotion

0.1 % by weight ofpLTA or pLTA analogue
1.70% by weight of sitosterol
1.50% by weight of polyglyceryl 2-oleate
1.2% by weight of ceteareth-4
1.5% by weight of cholesterol
0.4% by weight of dicetylphosphate
5.0% by weight of concentrated glycerin
10.0% by weight of sunflower oil
0.2% by weight of carboxyvinyl polymer
0.3% by weight of xanthan gum
Trace amount of antiseptic
Trace amount of spice
Balance of distilled water

### Preparative Example 5: Adjuvant including pLTA

### Preparation of adjuvant including pLTA

25 µg of pLTA or pLTA analogue
500 µg of dioleoylphosphatidylcholine (DOPC)
125 µg of cholesterol
Balance of a phosphate NaCl buffer solution and water
Sum: 0.5 ml

An adjuvant including the above-described components was prepared as described in WO96/33739

**Table 3**

| **Preparation of influenza vaccine compositions including pLTA adjuvant** | |
|---|---|
| **Components** | **Amount per single dose** |
| **Active components** | |
| **Inactivated split virion** | |
| - A/New Caledonia/20/99 (H1N1) | 15 µg HA |
| IVR-116 | |
| - A/New York/55/2004 (H3N2) | 15 µg HA |
| NYMC X-157 | |
| - B/Jiangsu/10/2003 | 15 µg HA |

| adjuvant | |
|---|---|
| - Liposome | |
| • Dioleoylphosphatidylcholine (DOPC) | 1000 µg |
| • Cholesterol | 250 µg |
| pLTA or pLTA analogue | 50 µg |

A single dose of the influenza vaccine corresponds to 1 ml.

## Claims

1. A glycolipid having 2 to 6 saturated or unsaturated C₁₀ to C₃₀ acyl chains bound to at least one of C₁, C₃, C₄ and C₆ positions dihexose or trihexose which is linked by an α-bond.

2. The glycolipid of claim 1, wherein the glycolipid has a structure represented by the following Formula I: 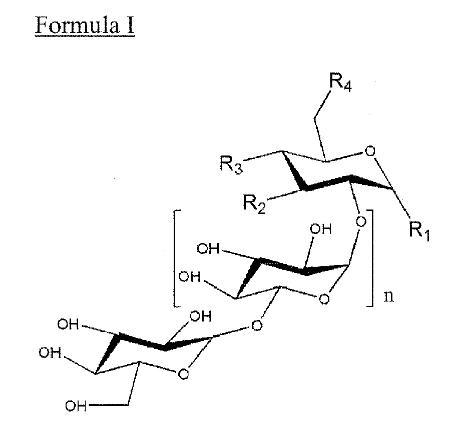 wherein R₁ to R₄ each independently represent a hydroxyl group, 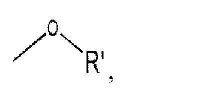 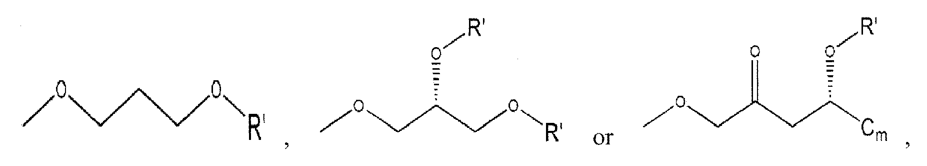 provided that R' each independently represents a saturated or unsaturated C₁₀ to C₃₀ acyl chain;
n is 0 or 1;
Cₘ represents a C₄ to C₃₀ alkyl group; and
the total number of the acyl chains in the glycolipid is in a range of 2 to 6.

3. The glycolipid of claim 2, wherein R₁ represents 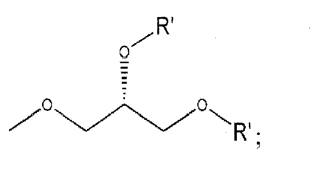 provided that R' each independently represents a saturated or unsaturated C₁₀ to C₃₀ acyl chain;
R₂ to R₄ each independently represent a hydroxyl group; and
n is 1,
wherein the glycolipid includes at least one acyl chain having 1 to 14 double bonds.

4. The glycolipid of claim 2, wherein R₁ represents 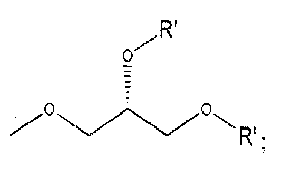 one of R₂, R₃ and R₄ represents 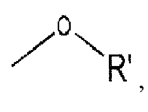 and the others represent a hydroxyl group, provided that R' each independently represents a saturated or unsaturated C₁₀ to C₃₀ acyl chain; and
n is 1,
wherein the glycolipid includes at least one acyl chain having 1 to 14 double bonds.

5. The glycolipid of claim 2, wherein Formula I is the following Formula I-1 or I-2:

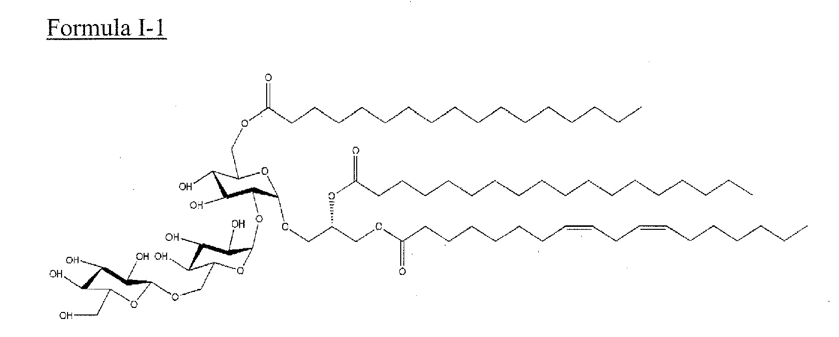

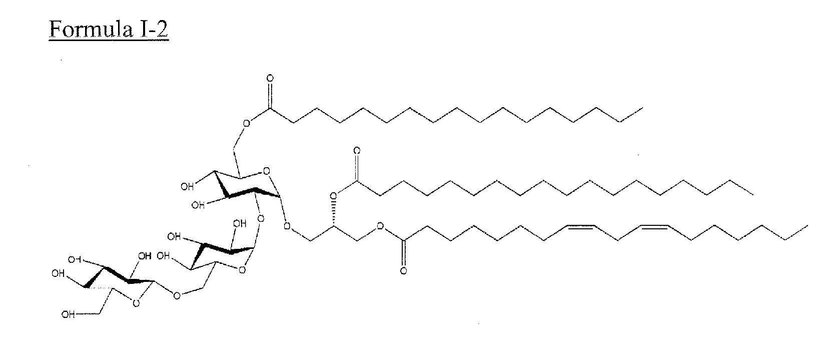

6. The glycolipid of claim 2, wherein the glycolipid is derived from a lactobacillus.

7. The glycolipid of claim 6, wherein the lactobacillus is from the genus *Lactobaccillus, Bifidobacterium, Streptococcus* or *Lactococcus.*

8. The glycolipid of claim 7, wherein the lactobacillus is *Lactobacillus plantarum, Lactobacillus rhamnosus, Lactobacillus delbrueckii, Lactobacillus acidophilus, Lactobacillus gasseri, Lactobacillus johnsonni, Lactobacillus helveticus, Lactobacillus casei, Bifidobacterium bifido, Bifidobacterium longum, Bifidobacterium infantis, Bifidobacterium animalis, streptococcus thermophilus or Lactococcus lactis.*

9. The glycolipid of claim 1, wherein poly(glycerophosphate) is further bound to a C₆ position of the hexose to which the acyl chain is not bound.

10. The glycolipid of claim 9, wherein the glycolipid has a structure represented by the following Formula II: 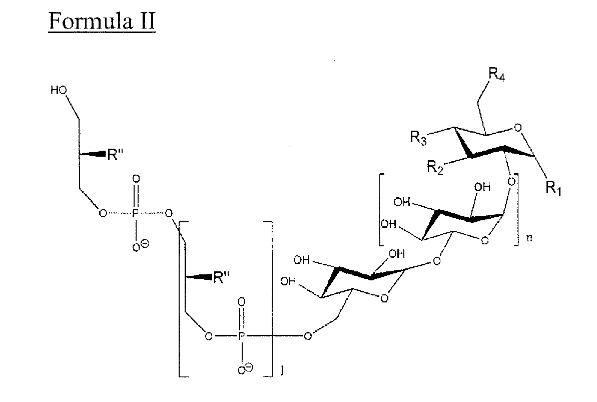 wherein R₁ to R₄ each independently represent a hydroxyl group, 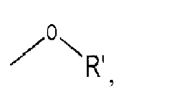 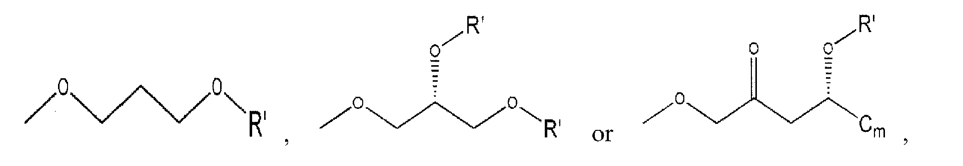 provided that R' each independently represents a saturated or unsaturated C₁₀ to C₃₀ acyl chain;
R" each independently represents an N-acetylglucosamine, D-alanine or hydroxyl group;
n is 0 or 1;
Cₘ represents a C₄ to C₃₀ alkyl group;
1 is in a range of 0 to 80; and
the total number of the acyl chains in the glycolipid is in a range of 2 to 6.

11. The glycolipid of claim 10, wherein R₁ represents 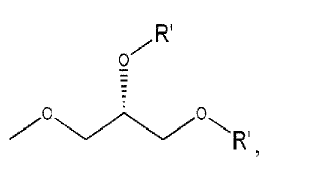 provided that R' each independently represents a saturated or unsaturated C₁₀ to C₃₀ acyl chain;
R" each independently represents an N-acetylglucosamine, D-alanine or hydroxyl group;
R₂ to R₄ each independently represent a hydroxyl group;
n is 1; and
1 is in a range of 0 to 80,
wherein the glycolipid includes at least one acyl chain having 1 to 14 double bonds.

12. The glycolipid of claim 10, wherein R₁ represents 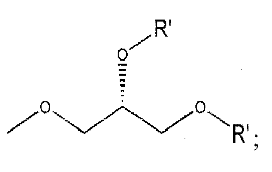 one of R₂, R₃ and R₄ represents 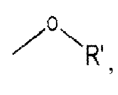 and the others represent a hydroxyl group, provided that R' each independently represents a saturated or unsaturated C₁₀ to C₃₀ acyl chain;
R" each independently represents an N-acetylglucosamine, D-alanine or hydroxyl group;
n is 1; and
1 is in a range of 0 to 80;
wherein the glycolipid includes at least one acyl chain having 1 to 14 double bonds.

13. The glycolipid of claim 10, wherein Formula II is the following Formula II-1 or II-2: 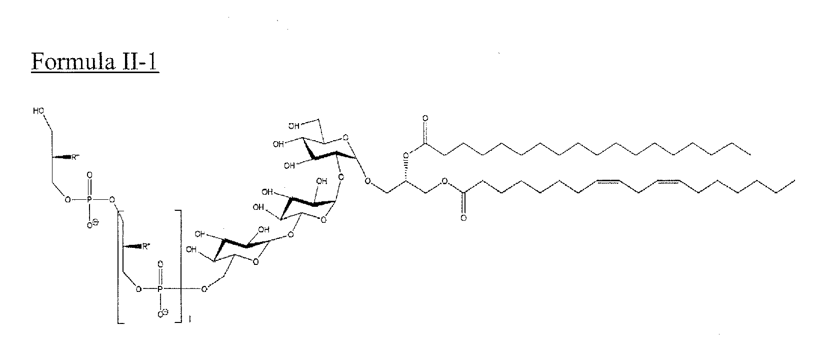 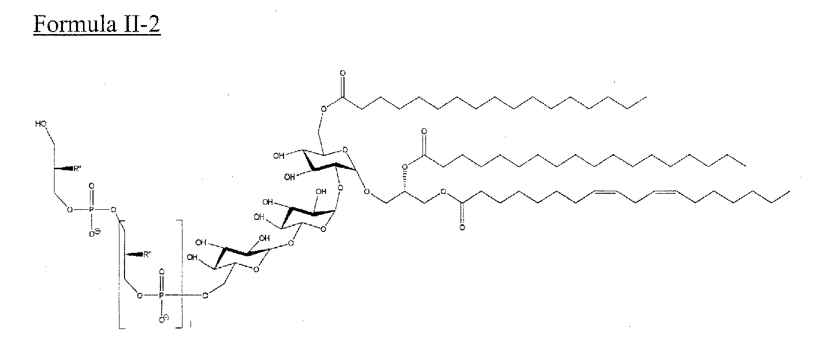 wherein R" each independently represents an N-acetylglucosamine, D-alanine or hydroxyl group; and
1 is in a range of 0 to 80.

14. The glycolipid of claim 9, wherein the glycolipid is a lactobacillus-derived lipoteichoic acid (LTA).

15. The glycolipid of claim 10, wherein the lactobacillus is from the genus *Lactobacillus, Bifidobacterium, Streptococcus* or *Lactococcus.*

16. The glycolipid of claim 10, wherein the lactobacillus is *Lactobacillus plantarum, Lactobacillus rhamnosus, Lactobacillus delbrueckii, Lactobacillus acidophilus, Lactobacillus gasseri, Lactobacillus johnsonni, Lactobacillus helveticus, Lactobacillus casei, Bifidobacterium bifido, Bifidobacterium longum, Bifidobacterium infantis, Bifidobacterium animalis, streptococcus thermophilus or Lactococcus lactis.*

17. A pharmaceutical composition comprising the glycolipid defined in any one of claims 1 to 16.

18. The pharmaceutical composition of claim 17, further comprising at least one additive selected from the group consisting of an antibiotic, a carrier, an excipient and a diluent.

19. The pharmaceutical composition of claim 17, wherein the pharmaceutical composition is formulated into an oral formulation, an external preparation, a suppository or a sterile injectable solution in the form of a powder, granule, tablet, capsule, suspension, emulsion, syrup or aerosol.

20. The pharmaceutical composition of claim 17, wherein the glycolipid is included at a content of 0.01 to 99.9 parts by weight, based on 100 parts by weight of the pharmaceutical composition.

21. A pharmaceutical composition for preventing and treating an inflammatory or immune-related disease, comprising the glycolipid defined in any one of claims 1 to 16.

22. The pharmaceutical composition of claim 21, wherein the inflammatory disease or immune-related disease is at least one selected from the group consisting of septicemia, arteriosclerosis, bacteremia, a systemic inflammatory response syndrome, a multiple organ dysfunction syndrome, cancer, osteoporosis, paradentitis, systemic lupus erythematosus, rheumatoid arthritis, osteoarthritis, juvenile chronic arthritis, spondyloarthropathy, systemic scleroma, an idiopathic inflammatory disorder of muscle, Sjögren's syndrome, systemic vasculitis, sarcoidosis, autoimmune hemolytic anemia, autoimmune thrombocytopenia, thyroiditis, diabetes mellitus, an immune-mediated renal disease, a demyelinating disease in the central nervous system or peripheral nervous system, idiopathic demyelinating polyneuropathy, Guillain-Barre syndrome, chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease (IBD), ulcerative colitis, Crohn's disease, irritable bowel syndrome, gluten-irritable bowel disease, Whipple's disease, an autoimmune or immune-mediated skin disease, a bullous skin disease, erythema multiforme, contact dermatitis, psoriasis, an allergic disease, asthma, allergic rhinitis, atopic dermatitis, food sensitivity, urticaria, a pulmonary immune disease, eosinophilic pneumonia, idiopathic pulmonary fibrosis, hypersensitivity pneumonitis, a transplant-related disease, a transplant rejection and a graft-versus-host disease.

23. The pharmaceutical composition of claim 21, wherein the pharmaceutical composition is administered orally, rectally or by intravenous, intramuscular, subcutaneous, endometrial or intracerebroventricular injection.

24. The pharmaceutical composition of claim 21, wherein the glycolipid is administered daily at a dose of 0.01 to 10,000 mg/kg.

25. A food composition comprising the glycolipid defined in any one of claims 1 to 16.

26. The food composition of claim 25, further comprising at least one additive selected from the group consisting of an organic acid, a phosphate, an antioxidant, lactose casein, dextrin, dextrose, a sugar and sorbitol.

27. The food composition of claim 25, wherein the glycolipid is included at a content of 0.01 to 99.9 parts by weight, based on 100 parts by weight of the food composition.

28. The food composition of claim 25, wherein the food composition is formulated into a solid, powder, granule, tablet, capsule or liquid type.

29. A cosmetic composition comprising the glycolipid defined in any one of claims 1 to 16.

30. The cosmetic composition of claim 29, further comprising at least one additive selected from the group consisting of a vitamin, an amino acid, a protein, a surfactant, an emulsifying agent, a spice, a pigment, a stabilizing agent, an antiseptic, an antioxidant, a UV blocking agent, a pH controlling agent and a chelating agent.

31. An adjuvant consisting of or including the glycolipid defined in any one of claims 1 to 16.
